(19) Europäisches Patentamt
European Patent Office
Office européen des brevets

(11) **EP 2 062 974 B1**

(12) **EUROPEAN PATENT SPECIFICATION**

(45) Date of publication and mention of the grant of the patent:
**12.08.2015 Bulletin 2015/33**

(21) Application number: **07792777.0**

(22) Date of filing: **21.08.2007**

(51) Int Cl.:
***C12N 15/09*** *(2006.01)* ***C07K 16/00*** *(2006.01)*
***C07K 19/00*** *(2006.01)* ***C12N 1/21*** *(2006.01)*
***C12P 21/02*** *(2006.01)*

(86) International application number:
**PCT/JP2007/066165**

(87) International publication number:
**WO 2008/023689 (28.02.2008 Gazette 2008/09)**

# (54) METHOD OF PRODUCING FUSED PROTEIN

VERFAHREN ZUR HERSTELLUNG VON FUSIONSPROTEIN

PROCÉDÉ DE PRODUCTION D'UNE PROTÉINE DE FUSION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **21.08.2006 JP 2006224657**

(43) Date of publication of application:
**27.05.2009 Bulletin 2009/22**

(73) Proprietors:
- **National University Corporation Kobe University**
  **Kobe-shi, Hyogo 657-8501 (JP)**
- **CHUGAI SEIYAKU KABUSHIKI KAISHA**
  **Tokyo, 115-8543 (JP)**

(72) Inventors:
- **KATO, Shigeo**
  **Kyoto-shi**
  **Kyoto 6068307 (JP)**
- **KUMADA, Yoichi**
  **Sakyo-ku**
  **Kyoto 606-8585 (JP)**
- **KAWASAKI, Tomomi**
  **Nishinomiya-shi**
  **Hyogo 6620831 (JP)**
- **KIKUCHI, Yasufumi**
  **Gotemba-shi**
  **Shizuoka 4128513 (JP)**

(74) Representative: **Woods, Geoffrey Corlett**
**J A Kemp**
**14 South Square**
**Gray's Inn**
**London WC1R 5JJ (GB)**

(56) References cited:
**WO-A1-03/085114 WO-A1-2006/106903**
**WO-A2-01/68835 US-A- 5 082 767**

- **KOMAR ANTON A ET AL: "Enhanced expression of the yeast Ure2 protein in Escherichia coli: The effect of synonymous codon substitutions at a selected place in the gene" BIOLOGICAL CHEMISTRY, WALTER DE GRUYTER GMBH & CO, BERLIN, DE, vol. 379, no. 10, 1 October 1998 (1998-10-01), pages 1295-1300, XP008120950 ISSN: 1431-6730**
- **KOMAR A A ET AL: "Synonymous codon substitutions affect ribosome traffic and protein folding during in vitro translation" FEBS LETTERS, ELSEVIER, AMSTERDAM, NL LNKD-DOI:10.1016/S0014-5793(99)01566-5, vol. 462, no. 3, 3 December 1999 (1999-12-03), pages 387-391, XP004260651 ISSN: 0014-5793**
- **RAMACHANDIRAN V ET AL: "Single synonymous codon substitution eliminates pausing during chloramphenicol acetyl transferase synthesis on Escherichia coli ribosomes in vitro" FEBS LETTERS, ELSEVIER, AMSTERDAM, NL LNKD- DOI: 10.1016/S0014-5793(02)02261-5, vol. 512, no. 1-3, 13 February 2002 (2002-02-13), pages 209-212, XP004341379 ISSN: 0014-5793**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SÁNCHEZ GLÒRIA ET AL: "Genome variability and capsid structural constraints of hepatitis a virus." JOURNAL OF VIROLOGY JAN 2003, vol. 77, no. 1, January 2003 (2003-01), pages 452-459, XP002576154 ISSN: 0022-538X
- GUISEZ Y ET AL: "Folding of the MS2 coat protein in Escherichia coli is modulated by translational pauses resulting from mRNA secondary structure and codon usage: a hypothesis." JOURNAL OF THEORETICAL BIOLOGY 21 MAY 1993, vol. 162, no. 2, 21 May 1993 (1993-05-21), pages 243-252, XP002576155 ISSN: 0022-5193
- SUGAHARA T ET AL: "Expression of biologically active fusion genes encoding the common [alpha] subunit and the follicle-stimulating hormone [beta] subunit: Role of a linker sequence" JOURNAL OF BIOLOGICAL CHEMISTRY 1996 US, vol. 271, no. 18, 1996, pages 10445-10448, XP002576156 ISSN: 0021-9258
- CAMEEL H MAKOUL ET AL: "Distribution of rare triplets along mRNA and their relation to protein folding" JOURNAL OF BIOMOLECULAR STRUCTURE & DYNAMICS, ADENINE PRESS, NEW YORK, NY, US, vol. 20, no. 3, 1 December 2002 (2002-12-01), pages 413-420, XP008120893 ISSN: 0739-1102
- WU G ET AL: "The Synthetic Gene Designer: A flexible web platform to explore sequence manipulation for heterologous expression" PROTEIN EXPRESSION AND PURIFICATION, ACADEMIC PRESS, SAN DIEGO, CA LNKD- DOI: 10.1016/J.PEP.2005.10.020, vol. 47, no. 2, 1 June 2006 (2006-06-01), pages 441-445, XP024908763 ISSN: 1046-5928 [retrieved on 2006-06-01]
- ANGOV E ET AL: "Overcoming obstacles in protein expression using codon harmonization" PARASITE IMMUNOLOGY, BLACKWELL SCIENTIFIC PUBLICATIONS, OXFORD, GB, vol. 28, no. 6, 1 June 2006 (2006-06-01), page 250, XP009125585 ISSN: 0141-9838
- KUMADA ET AL: "Polypeptide linkers suitable for the efficient production of dimeric scFv in Escherichia coli" BIOCHEMICAL ENGINEERING JOURNAL, ELSEVIER, AMSTERDAM, NL, vol. 35, no. 2, 10 May 2007 (2007-05-10), pages 158-165, XP022069080 ISSN: 1369-703X
- KUMADA Y ET AL: "Effect of nucleotide sequences of polypeptide linkers on production of soluble single-chain Fv antibodies" JOURNAL OF CHEMICAL ENGINEERING OF JAPAN, SOCIETY OF CHEMICAL ENGINEERS, JP, vol. 41, no. 5, 1 January 2008 (2008-01-01), pages 413-419, XP008120744 ISSN: 0021-9592
- KUMADA Y ET AL: "Efficient production of single-chain Fv antibody possessing rare codon linkers in fed-batch fermentation" JOURNAL OF BIOSCIENCE AND BIOENGINEERING, ELSEVIER, AMSTERDAM, NL, vol. 107, no. 1, 1 January 2009 (2009-01-01), pages 73-77, XP025880589 ISSN: 1389-1723 [retrieved on 2009-01-13]
- KOMAR A.A. ET AL.: 'Kinetics of translation of ganmaB crystalline and its circularly permutated variant in an in vitro cell-free system: possible relations to codon distribution and protein folding' FEBS LETTERS vol. 376, 1995, pages 195 - 198, XP003021222
- SMALLSHAW J.E. ET AL.: 'Synthesis, cloning and expression of the single-chain Fv gene of the HPr-specific monoclonal antibody, Jel42. Determination of binding constants with wild-type and mutant HPrs' PROTEIN ENGINEERING vol. 12, no. 7, 1999, pages 623 - 630, XP003021223
- HUSTON J.S. ET AL.: 'Protein engineering of antibody binding sites: Recovery of specific activity in an anti-digoxin single-chain Fv analogue produced in Escherichia coli' PROC. NATL. ACAD. SCI. USA vol. 85, no. 16, 1988, pages 5879 - 5883, XP000872837
- KAWASAKI T. ET AL.: 'Polypeptide linkers no Kaihen ni yoru Ipponsa Kotai no Koritsuteki Seisan' THE SOCIETY OF CHEMICAL ENGINEERS, JAPAN NENKAI KENKYU HAPPYO KOEN YOSHISHU February 2007, page 347, XP003021224
- KUMADA Y. ET AL.: 'Polypeptide linkers suitable for the efficient production of dimeric scFV in Escherichia coli' BIOCHEMICAL ENGINEERING JOURNAL vol. 35, no. 2, July 2007, pages 158 - 165, XP022069080
- KUMADA Y. ET AL.: '1 Honsa Kotai Seisan ni okeru Polypeptide linkers no Oyobosu Eikyo' THE SOCIETY OF CHEMICAL ENGINEERS, JAPAN NENKAI KENKYU HAPPYO KOEN YOSHISHU 2005, page 308, XP003021225
- KUMADA Y. ET AL.: '1 Honsa Kotai Seisan ni Oyobosu Polypeptide linkers no Eikyo' THE SOCIETY OF CHEMICAL ENGINEERS, JAPAN SHUKI TAIKAI KENKYU HAPPYO KOEN YOSHISHU 2005, page W107, XP003021226
- TURNER D.J. ET AL.: 'Importance of the linker in expression of single-chain Fv antibody fragments: optimization of peptide sequence using phage display technology' JOURNAL OF IMMUNOLOGICAL METHODS vol. 205, no. 1, 1997, pages 43 - 54, XP004082109
- LILJENSTROM H. ET AL.: 'Translation Rate Modification By Preferential Codon Usage: Intragenic Position Effects' J. THEOR. BIOL. vol. 124, no. 1, 1987, pages 43 - 55, XP009092743
- SORENSEN M.A. ET AL.: 'Codon Usage Determines Translation Rate in Escherichia coli' J. MOL. BIOL. vol. 207, 1989, pages 365 - 377, XP003021227
- IVANOV I.G. ET AL.: 'Unusual Effect of Clusters of Rate Arginine (AGG) Codons on the Expression of Human Interferon alpha 1 Gene in Escherichia coli' INT. J. BIOCHEM. CELL BIOL. vol. 29, no. 4, 1997, pages 659 - 666, XP003021228




(Cont. next page)

- CHEN T.G.-F. ET AL.: 'Suppression of the negative effect of minor arginine codons on gene expression; preferential usage of minor codons within the first 25 codons of the Escherichia coli genes' NUCLEIC ACIDS RESEARCH vol. 18, no. 6, 1990, pages 1465 - 1473, XP003021229
- WOO JUNG HEE ET AL: "Gene optimization is necessary to express a bivalent anti-human anti-T cell immunotoxin in Pichia pastoris", PROTEIN EXPRESSION AND PURIFICATION, ACADEMIC PRESS, SAN DIEGO, CA, vol. 25, no. 2, 1 July 2002 (2002-07-01), pages 270-282, XP002266044, ISSN: 1046-5928, DOI: DOI:10.1016/S1046-5928(02) 00009-8
- HUSTON J S ET AL: "Protein engineering of single-chain Fv analogs and fusion proteins", METHODS IN ENZYMOLOGY; [METHODS IN ENZYMOLOGY], ACADEMIC PRESS INC, SAN DIEGO, CA, US, vol. 203, 1 January 1991 (1991-01-01), pages 46-88, XP008120115, ISSN: 0076-6879
- TAKKINEN K ET AL: "An active single-chain antibody containing a cellulase linker domain is secreted by Escherichia coli", PROTEIN ENGINEERING, OXFORD UNIVERSITY PRESS, SURREY, GB, vol. 4, no. 7, 1 October 1991 (1991-10-01) , pages 837-841, XP008138112, ISSN: 0269-2139, DOI: DOI:10.1093/PROTEIN/ 4.7.837
- VERMA R ET AL: "Antibody engineering: Comparison of bacterial, yeast, insect and mammalian expression systems", JOURNAL OF IMMUNOLOGICAL METHODS, ELSEVIER SCIENCE PUBLISHERS B.V.,AMSTERDAM, NL, vol. 216, no. 1-2, 1 July 1998 (1998-07-01), pages 165-181, XP004143134, ISSN: 0022-1759, DOI: DOI:10.1016/S0022-1759(98)00077-5
- MEHDI ARBABI-GHAHROUDI ET AL: "Prokaryotic expression of antibodies", CANCER AND METASTASIS REVIEWS, KLUWER ACADEMIC PUBLISHERS, DO, vol. 24, no. 4, 1 December 2005 (2005-12-01), pages 501-519, XP019205203, ISSN: 1573-7233, DOI: DOI: 10.1007/S10555-005-6193-1
- TRINH R ET AL: "Optimization of codon pair use within the (GGGGS)3 linker sequence results in enhanced protein expression", MOLECULAR IMMUNOLOGY, PERGAMON, GB, vol. 40, no. 10, 1 January 2004 (2004-01-01), pages 717-722, XP002495823, ISSN: 0161-5890, DOI: DOI: 10.1016/J.MOLIMM.2003.08.006

SEQUENCE LISTING

<110> Kobe University
<120> Method for preparing fusion protein
<130> PCG-9017WO
<150> JP 2006-224657
<151> 2006-08-21

<160> 38
<170> PatentIn version 3.1
<210> 1
<211> 20
<212> PRT
<213> artificial sequence
<220>
<223> artificial linker
<400> 1

```
Gly Met Gly Leu Ser Trp Ala Gly Gln Leu Pro Arg Phe Pro Gln Arg
1               5                   10                  15
Ala Ser Gln Gly
            20
```

<210> 2
<211> 60
<212> DNA
<213> artificial sequence
<220>
<223> artificial linker
<400> 2
ggtatgggtt tgtcttgggc tgggcagctg cctaggtttc ctcagcgtgc tagtcaaggg 60
<210> 3
<211> 20
<212> PRT
<213> artificial sequence
<220>
<223> artificial linker
<400> 3

```
Lys Gly Arg Gln Gln Leu Gln Leu Cys Ala Pro Phe Asp Trp Cys Met
1               5                   10                  15
Phe Asp Ala Asn
            20
```

<210> 4
<211> 60
<212> DNA
<213> artificial sequence
<220>
<223> artificial linker
<400> 4
aagggtcggc aacagctgca gttgtgtgcg ccttttgatt ggtgtatgtt tgatgctaat 60
<210> 5
<211> 20
<212> PRT
<213> artificial sequence
<220>
<223> artificial linker
<400> 5

```
Trp Val Trp Ser Ser Arg Gly Gln Arg Ser Phe Arg Pro Ser Gly Arg
1               5                   10                  15
Thr Val Pro Leu
            20
```

<210> 6

<211> 60
<212> DNA
<213> artificial sequence
<220>
<223> artificial linker
<400> 6
tgggtttgga gttcgcgggg gcagaggtct tttcggcctt cggggcggac ggtgccgctt 60
<210> 7
<211> 20
<212> PRT
<213> artificial sequence
<220>
<223> artificial linker
<400> 7

```
Lys Val Val Leu Trp Thr Thr Arg Val Arg Asp Arg Gly His Thr Ser
1               5                   10                  15
Thr Met Trp Ser
            20
```

<210> 8
<211> 60
<212> DNA
<213> artificial sequence
<220>
<223> artificial linker
<400> 8
aaggttgttc tttggactac gcgtgttagg gataggggtc atacgtcgac gatgtggagt 60
<210> 9
<211> 20
<212> PRT
<213> artificial sequence
<220>
<223> artificial linker
<400> 9

```
Ala Asp Gly His Cys His Leu Lys Asn Phe Pro Leu Lys Pro Pro Pro
1               5                   10                  15
Tyr Phe Ser Val
            20
```

<210> 10
<211> 60
<212> DNA
<213> artificial sequence
<220>
<223> artificial linker
<400> 10
gcggatgggc attgtcatct gaagaatttt cctttgaagc ctccgcctta tttttcggtt 60
<210> 11
<211> 16
<212> PRT
<213> artificial sequence
<220>
<223> artificial linker
<400> 11

Leu Leu Lys Lys Leu Leu Lys Lys Leu Leu Lys Lys Leu Leu Lys Lys
1 5 10 15

<210> 12
<211> 48
<212> DNA
<213> artificial sequence
<220>
<223> artificial linker
<400> 12
ctactaaaaa aactactaaa aaaactacta aaaaaactac taaaaaaa 48
<210> 13
<211> 15
<212> PRT
<213> artificial sequence
<220>
<223> artificial linker
<400> 13

Gly Gly Gly Gly Ser Gly Gly Gly Gly Ser Gly Gly Gly Gly Ser
1 5 10 15

<210> 14
<211> 45
<212> DNA
<213> artificial sequence
<220>
<223> artificial linker
<400> 14
ggtggaggcg gttccggcgg aggtggctcc ggcggtggcg gatcc 45
<210> 15
<211> 119
<212> PRT
<213> Mus musculus
<400> 15

Asp Val Gln Leu Gln Glu Ser Gly Pro Gly Leu Val Lys Pro Ser Gln
1 5 10 15
Ser Leu Ser Leu Thr Cys Ser Val Thr Gly Tyr Ser Ile Thr Ser Gly
20 25 30
Tyr Tyr Trp Asn Trp Ile Arg Gln Phe Pro Gly Asn Lys Leu Glu Trp
35 40 45
Met Gly Tyr Ile Arg Tyr Asp Gly Ser Asn Asn Tyr Asn Pro Ser Leu
50 55 60
Lys Asn Arg Ile Ser Ile Thr Arg Asp Thr Ser Lys Asn Gln Phe Phe
65 70 75 80
Leu Lys Leu Asn Ser Val Thr Pro Glu Asp Thr Ala Thr Tyr Tyr Cys
85 90 95
Ala Arg Val Leu Gly Arg Gly Tyr Gly Leu Asp Tyr Trp Gly Gln Gly
100 105 110
Thr Ser Val Thr Val Ser Ser
115

<210> 16
<211> 357
<212> DNA
<213> Mus musculus
<400> 16

```
gatgtacagc ttcaggagtc aggacctggc ctcgtgaaac cttctcagtc tctgtctctc     60
acctgctctg tcactggcta ctccatcacc agtggttatt actggaactg gatccggcag    120
tttccaggaa acaaactgga atggatgggc tatataaggt acgacggtag caataactac    180
aacccatctc tcaaaaatcg aatctccatc actcgtgaca catctaagaa ccagtttttc    240
ctgaaattga attctgtgac tcctgaggac acagctacat attactgtgc aagagtattg    300
ggacggggct atggtttgga ctactggggt caaggaacct cagtcaccgt ctcctca       357
```

<210> 17
<211> 111
<212> PRT
<213> Mus musculus
<400> 17

```
Asp Ile Val Leu Thr Gln Ser Pro Ala Ser Leu Ala Val Ser Leu Gly
1               5                   10                  15
Gln Arg Ala Thr Ile Ser Cys Arg Ala Ser Gln Ser Val Ser Thr Ser
            20                  25                  30
Thr Tyr Ser Tyr Leu His Trp Tyr Gln Gln Arg Pro Gly Gln Pro Pro
        35                  40                  45
Lys Leu Ile Lys Tyr Val Ser Asn Leu Glu Ser Gly Val Pro Ala Arg
    50                  55                  60
Phe Ser Gly Ser Gly Ser Gly Thr Asp Phe Thr Leu Asn Ile His Pro
65                  70                  75                  80
Val Glu Glu Glu Asp Thr Ala Thr Tyr Tyr Cys Gln His Ser Trp Glu
                85                  90                  95
Ile Pro Pro Thr Phe Gly Gly Gly Thr Lys Leu Glu Ile Lys Arg
            100                 105                 110
```

<210> 18
<211> 333
<212> DNA
<213> Mus musculus
<400> 18

```
gacattgtgc tgacacagtc tcctgcttcc ttagctgtat ctctggggca gagggccacc     60
atctcatgca gggccagcca aagtgtcagt acatctacct atagttattt acactggtac    120
caacagagac caggacagcc acccaaactc atcaagtatg tatccaacct agaatctggg    180
gtccctgcca ggttcagtgg cagtgggtct gggacagact tcaccctcaa catccatcct    240
gtggaggagg aggatactgc aacatattac tgtcagcaca gttgggagat tcctccgacg    300
ttcggtggag gcaccaagct ggaaatcaaa cgg                                 333
```

<210> 19
<211> 94
<212> DNA
<213> artificial sequence
<220>
<223> artificial linker
<220>
<221> misc_feature
<222> (18)..(19)
<223> n is any nucleotide
<220>
<221> misc_feature
<222> (21)..(22)
<223> n is any nucleotide
<220>
<221> misc_feature

<222> (24)..(25)
<223> n is any nucleotide
<220>
<221> misc_feature
<222> (27)..(28)
<223> n is any nucleotide
<220>
<221> misc_feature
<222> (30)..(31)
<223> n is any nucleotide
<220>
<221> misc_feature
<222> (33)..(34)
<223> n is any nucleotide
<220>
<221> misc_feature
<222> (36)..(37)
<223> n is any nucleotide
<220>
<221> misc_feature
<222> (39)..(40)
<223> n is any nucleotide
<220>
<221> misc_feature
<222> (42)..(43)
<223> n is any nucleotide
<220>
<221> misc_feature
<222> (45)..(46)
<223> n is any nucleotide
<220>
<221> misc_feature
<222> (48)..(49)
<223> n is any nucleotide
<220>
<221> misc_feature
<222> (51)..(52)
<223> n is any nucleotide
<220>
<221> misc_feature
<222> (54)..(55)
<223> n is any nucleotide
<220>
<221> misc_feature
<222> (57)..(58)
<223> n is any nucleotide
<220>
<221> misc_feature
<222> (60)..(61)
<223> n is any nucleotide
<220>
<221> misc_feature
<222> (63)..(64)
<223> n is any nucleotide
<220>
<221> misc_feature
<222> (66)..(67)
<223> n is any nucleotide

<220>
<221> misc_feature
<222> (69)..(70)
<223> n is any nucleotide
<220>
<221> misc_feature
<222> (72)..(73)
<223> n is any nucleotide
<220>
<221> misc_feature
<222> (75)..(76)
<223> n is any nucleotide
<400> 19

cagtcaccgt ctcctcannk nnknnknnkn nknnknnknn knnknnknnk nnknnknnkn 60
nknnknnknn knnknnkgac attgtgctga caca 94

<210> 20
<211> 41
<212> DNA
<213> artificial sequence
<220>
<223> artificial linker
<400> 20
ctcccatggc cgatgtacag cttcaggagt caggacctgc c 41
<210> 21
<211> 37
<212> DNA
<213> artificial sequence
<220>
<223> artificial linker
<400> 21
atatatgcgg ccgcccgttt gatttccagc ttggtgc 37
<210> 22
<211> 30
<212> DNA
<213> artificial sequence
<220>
<223> artificial linker
<400> 22
tgaggagacg gtgactgagg ttccttgacc 30
<210> 23
<211> 30
<212> DNA
<213> artificial sequence
<220>
<223> artificial linker
<400> 23
gacattgtgc tgacacagtc tcctgcttcc 30
<210> 24
<211> 45
<212> DNA
<213> artificial sequence
<220>
<223> artificial linker
<400> 24
ggtggaggcg gttccggcgg aggtggctcc ggcggtggcg gatct 45

<210> 25
<211> 60
<212> DNA
<213> artificial sequence
<220>
<223> artificial linker
<400> 25
tgggtttgga gttcgcgtgg gcagcgttct tttcgtcctt cggggcgtac ggtgccgctt 60
<210> 26
<211> 24
<212> DNA
<213> artificial sequence
<220>
<223> PCR primer
<400> 26
caggtccagc tgcagcagtc tgga 24
<210> 27
<211> 27
<212> DNA
<213> artificial sequence
<220>
<223> PCR primer
<400> 27
tgaggagact gtgagagtgg tgccttg 27
<210> 28
<211> 30
<212> DNA
<213> artificial sequence
<220>
<223> PCR primer
<400> 28
gatgttgtga tgacccaaag tccactctcc 30
<210> 29
<211> 30
<212> DNA
<213> artificial sequence
<220>
<223> PCR primer
<400> 29
ttttatttcc agcttggtcc cccctccgaa 30
<210> 30
<211> 79
<212> DNA
<213> artificial sequence
<220>
<223> PCR primer
<400> 30

ctctcacagt ctcctcaggt ggaggcggtt caggcggagg tggctctggc ggtggcggat 60
cagatgttgt gatgaccca 79

<210> 31
<211> 79
<212> DNA
<213> artificial sequence
<220>
<223> PCR primer

<400> 31

ctctcacagt ctcctcaggt ggaggcggtt ccggcggagg tggctccggc ggtggcggat 60
ccgatgttgt gatgaccca 79

<210> 32
<211> 25
<212> DNA
<213> artificial sequence
<220>
<223> PCR primer
<400> 32
tttccatggc ccaggtccag ctgca 25
<210> 33
<211> 29
<212> DNA
<213> artificial sequence
<220>
<223> PCR primer
<400> 33
tttttgcgg ccgcttttat ttccagctt 29
<210> 34
<211> 52
<212> DNA
<213> artificial sequence
<220>
<223> PCR primer
<400> 34
tcatttatcg tcatcgtctt tgtagtcttt tatttccagc ttggtccccc ct 52
<210> 35
<211> 79
<212> DNA
<213> artificial sequence
<220>
<223> PCR primer
<400> 35

ccaagctgga aataaaaggt ggaggcggtt caggcggagg tggctctggc ggtggcggat 60
cacaggtcca gctgcagca 79

<210> 36
<211> 41
<212> DNA
<213> artificial sequence
<220>
<223> PCR primer
<400> 36
tttttgcgg ccgctcattt atcgtcatcg tctttgtagt c 41
<210> 37
<211> 79
<212> DNA
<213> artificial sequence
<220>
<223> PCR primer
<400> 37

ccaagctgga aataaaaggt ggaggcggtt ccggcggagg tggctccggc ggtggcggat 60
cccaggtcca gctgcagca 79

<210> 38
<211> 48
<212> DNA
<213> artificial sequence
<220>
<223> artificial linker
<400> 38
ctactaaaaa aactactaaa aaaactacta aaaaaactac taaaaaaa 48

## Description

### TECHNICAL FIELD

The present invention relates to a process for producing a fusion protein through the use of genetic engineering techniques, wherein the fusion protein has a structure in which a plurality of polypeptide domains are joined by a polypeptide linker, and wherein the polypeptide linker is designed so that the translation rate is slower in the mRNA region corresponding to that polypeptide linker.

### BACKGROUND

The production of a large number of useful substances, particularly proteins, polypeptides, and the like, originating in biological organisms has become possible with the establishment of genetic engineering techniques. In the past, such substances originating in biological organisms were isolated by methods such as extraction, purification, and the like from a natural product containing the target substance as a starting material. In general, as the concentration of the target substance contained in the starting material is low, the yield of the target substance was limited. If genetic engineering techniques are used, however, it is possible to express a target protein, polypeptide, and the like in large quantities by transfecting a suitable host with a recombinant vector prepared by linking a gene encoding the protein, polypeptide, and the like to a genetic element capable of driving expression of the gene. Thus, it has become possible to obtain the target protein or polypeptide at a high yield by extraction or purification from a preparation containing a high concentration of the target protein or polypeptide obtained by large-quantity expression thereof.

Genetic engineering is a broad term for techniques that modify biological function of an organism by manipulating genes to impart a function that is different from the functions originally present in the organism. The industrial applications of genetic engineering encompass a broad range including medical therapy, analysis and diagnosis, agricultural and marine products, food products, and chemistry. More specifically a large number of products produced by genetic engineering have reached practical use as medicines, diagnostic agents, analytical reagents, recombinant animals, recombinant plants, enzymes, and the like. As noted above, genetic engineering is a technology for modifying biological function of an organism by manipulating genes to impart a function that is different from the functions originally present in the organism, namely, expressing in a host a gene that is not originally present in that host. Therefore, it is sometimes observed that the target substance cannot be expressed in its desired form in cases of heterologous gene expression where the species of the organism serving as the origin of the gene to be inserted (donor) is different from the species of the host organism in which the gene is to be expressed (recipient).

For example, when a glycoprotein originating in a eukaryotic organism is expressed in a prokaryotic organism such as *Escherichia coli* or *Bacillus subtilis,* which have been used for a long time as hosts for recombination, the produced recombinant protein does not have attached sugar chains because a mechanism for adding sugar chains is not present in prokaryotic cells. Therefore, if the presence of a sugar chain is essential for protein activity, the recombinant protein is produced as an inactive form of the protein.

As a similar example, it was known that the usage frequency of codons that are factors for determining the efficiency in gene transcription and translation, and that differences in the usage frequency of codons::among biological species may cause impaired expression. With the exception of eukaryotic organism mitochondria and *Candida* yeast, the amino acid codons are essentially shared by all organisms, but there is a difference in the codon usage frequency between eukaryotic organisms and prokaryotic organisms serving as hosts (Non-patent document 1: codon usage frequency database http://www.kazusa.or.jp/codon/). The following observations have been reported concerning the codon usage frequency.

1) The codon selection pattern is one important factor related to the amount of protein production. For example, when a gene originating in a eukaryotic organism that has been inserted into *Escherichia coli* uses Ile (AUA), Arg (AGA), Leu (CUA), Arg (AGG), Pro (CCC), and Gly (GGA), which have a low usage frequency in *E. coli,* either the protein encoded by that gene will not be expressed, or expression will terminate prematurely and the protein will undergo degradation. Even if the protein is expressed, granules (precipitate) solely containing large amounts of the foreign protein will be formed (hereinafter, such granules are referred to as inclusion body). Because the proteins in the inclusion bodies do not have their original three-dimensional conformation through intramolecular disulfide bond and the like, they will not have the desired activity without further processing.
2) The codon selection pattern is related not only to the amount of protein produced, but also to the accuracy of the transcription process. It was reported that a protein was produced with a different amino acid sequence that contains Lys (AAA) instead of Arg (AGA) (Non-patent document 2: J. Mol. Biol., 262, 407-412, 1996).
3) It is known that codon usage frequency in an organism and the concentration of the corresponding tRNA in that organism do not match perfectly. Therefore, to define a rare codon in an organism, it is necessary to consider not only the codon usage frequency, but also the tRNA concentration in that organism.

From the above observations it has been predicted that it is preferable to use synonymous codons that are often used in the host prokaryotic cells as codons for a gene originating in a eukaryotic organism. Therefore, in the process of chemical gene synthesis a gene is synthesized that has suitable codons based on the codon usage characteristics of the host bacteria (Non-patent document 3: Cell Technology [Saibo Kogaku], 5, 212-221, 1986). In addition, a host transfected with a recombinant plasmid that expresses tRNA corresponding to rare codons in *E. coli* (for example, CodonPlus™ (Stratagene) when using *E. coli* as a host) has been successfully developed. It has been reported that scFv were produced in *E. coli* transfected with tRNA corresponding to rare codons utilizing a similar technology, and that the amount of production was increased (Non-patent document 4: J. Biosci. Bioeng., 91(1), 53-7, 2001).

Examples of proteins originating in eukaryotic organisms that are frequently used in applications such as drugs, diagnostic agents, etc., include enzymes, and antibodies or fragments thereof. Enzymes are used in a wide range of fields such as food products, drugs, diagnostic agents, chemistry, and the like. Antibodies are used for diagnostic agents and drugs because of their high level of affinity with the antigens they recognize. Antibodies have come to be used as drugs in recent years with great frequency particularly because they are very stable in serum and have low antigenicity.

A full-length antibody molecule consists of two molecules each of two types of polypeptides called heavy chains an light chains, and a sugar chain structure is required for the activity of the antibody. Therefore, to produce an active form of an antibody molecule it is necessary to express the antibody in a recipient having the same sugar chain-addition activity as the donor eukaryotic cells. On the other hand, an antibody fragment is a fragment of a full-length antibody comprising a heavy-chain variable region (VH) and a light-chain variable region (VL) which are the minimal binding unit necessary for sufficient recognition of an antigen. An Fv molecule is a heterodimer consisting of VH and VL regions joined together. Another antibody fragment is a molecule wherein a VH polypeptide and an VL polypeptide are joined by a polypeptide linker of an arbitrary amino acid sequence. Antibody fragments have a shorter half-life in blood than the full-length antibody, and the concentration of an antibody fragment in blood may be artificially controlled by dosage. Moreover, the molecular weight of an antibody fragment is lower than the molecular weight of a full-length antibody, thus it has superior distribution profiles to tissues. Thus, an antibody fragment has excellent properties as a drug product compared to a full-length antibody. In addition, it has been reported that an antibody fragment may impart agonistic activity toward its receptor, which cannot be observed with a full-length IgG antibody (Non-patent document 5: Blood, 105(2), 562-6, 2005). Antibody fragments are promising as medicament from the standpoint of its functional superiority.

Because an antibody fragment does not require the addition of a sugar chain to function as an active form of the molecule, it is not necessary to express an antibody fragment in a recipient having the same sugar chain-addition activity as the donor eukaryotic cells. Therefore, it is possible to produce an antibody fragment with genetic engineering techniques in microorganisms such as *E. coli, B. subtilis* and the like (including eukaryotic organisms such as yeast, etc.). Such microorganisms are more suitable for manufacturing from the standpoint of productivity per unit time and convenience, due to their rapid growth rate and high production level.

However, when an antibody fragment is produced in *E. coli* and other prokaryotic host organisms, the antibody fragment lacking binding activity toward the antigen are frequently observed. This is because, just as in the case of most proteins originating in eukaryotic organisms, either the antibody fragment molecules aggregate and form an inclusion body or, even if they do not form an inclusion body, they do not form a suitable three-dimensional conformation. A method has been reported wherein the inclusion bodies are denatured under reducing conditions using guanidine hydrochloride or β-mercaptoethanol, and subsequently the denaturing reagent is gradually removed by dialysis. Then at the stage at which a soluble structure starts to form, the aggregation inhibitor arginine and oxidized glutathione, which promotes the formation of disulfide bonds, are added to promote refolding (Non-patent document 6: J. Immunol. Method, 219, 119-29, 1998). Because this procedure is complex and time consuming, development of a manufacturing process of an antibody fragment is demanded wherein an antibody fragment can be produced in a soluble form while retaining its binding activity.

The reference documents cited herein are listed below. However, none of these documents is admitted to be prior art of the present invention.

Non-patent document 1: codon usage frequency database http://www.kazusa.or.jp/codon/
Non-patent document 2: J. Mol. Biol., 262, 407-412, 1996
Non-patent document 3: Cell Technology [Saibo Kogaku], 5, 212-221, 1986
Non-patent document 4: J. Biosci. Bioeng., 91(1), 53-7,2001
Non-patent document 5: Blood, 105(2),562-6,2005
Non-patent document 6: J. Immunol. Method, 219,119-29,1998

The following documents are also acknowledged:

- Huston et al (1988) PNAS USA, 85(16): 5879-5883, which is concerned with protein engineering of antibody binding sites, in particular recovery of specific activity in an anti-digoxin single-chain Fv analogue produced in *Escherichia coli.*

- Hee et al (2002) Protein Expression and Purification, 25(2): 270-282, which is concerned with gene optimization as being necessary to express a bivalent anti-human anti-T cell immunotoxin in *Pichia pastoris.*
- Turner et al (1997) Journal of Immunological Methods, 205(1): 43-54, which is concerned with the importance of the linker in expression of single-chain Fv antibody fragments: optimisation of peptide sequence using phage display technology.
- Huston et al (1991) Methods in Enzymology 203: 46-88, which is concerned with protein engineering of single-chain Fv analogs and fusion proteins.
- Takkinen et al (1991) Protein Engineering, 4(7): 837-841, which is concerned with an active single-chain antibody containing a cellulose linker domain which is secreted by *Escherichia coli.*
- Verma et al (1998) Journal of Immunological Methods, 216(1-2): 165-181, which is concerned with antibody engineering, in particular comparison of bacterial, yeast, insect and mammalian expression systems.
- Arbabi-Ghahroudi et al (2005)c Cancer and metastasis Reviews, 24(4): 501-519, which is concerned with prokaryotic expression of antibodies
- Trinh et al (2004) Molecular Immunology, 40(10): 717-722, which is concerned with optimization of codon pair use within the (GGGGS)3 linker sequence results in enhanced protein expression.

DISCLOSURE OF THE INVENTION

An object of the present invention is to provide a process for producing an antibody fragment that can be produced in soluble form. A further object of the present invention is to provide a process for producing an antibody fragment that can be produced efficiently. Yet further object of the present invention is to provide a process for producing an antibody fragment that can be produced in a large quantity.

The inventors have discovered that the above objects are achieved by selecting a nucleotide sequence encoding a linker sequence used to join both functional domains of the antibody fragment together to achieve the present invention.

The present invention provides a process for producing an intracellular soluble fraction of an antibody fragment, where the antibody fragment comprises variable domains joined by a polypeptide linker or linkers which consist of 5 to 50 amino acids, said method comprising:

(a) introducing, in the polynucleotide sequence encoding the linker(s), one or more rare codons, where a rare codon is one which has a tRNA ratio of 1.2% or less of the total tRNA in a prokaryotic host cell; and
(b) culturing the prokaryotic host cell, which has been transformed by a vector comprising the polynucleotide encoding the antibody fragment inserted into the vector to allow the polynucleotide to be expressed in the host cell, and recovering the antibody fragment thus produced,

where the sequence of the polynucleotide encoding the polypeptide linker(s) is such that when the mRNA transcribed from the polynucleotide is translated in the host cell transfected with the polynucleotide, the translation rate of the mRNA region encoding the polypeptide linker(s) is slower than the translation rate of the mRNA region encoding the variable domain immediately upstream thereof. In other words, the polynucleotide sequence encoding the polypeptide linker is selected such that the translation rate of the linker sequence is delayed when the mRNA generated by transcription of the nucleotide sequence is translated.

The polynucleotide encoding the polypeptide linker contains one or more rare codons, where a rare codon is one which has a tRNA ratio of 1.2% or less of the total tRNA in a prokaryotic cell. More preferably, the rare codon is selected from any of GCC, CGG, AGG, CAA, CAC, CAT, CTA, CCC, CCA, and TCC. Even more preferably, the rare codon is selected from any of CGG, AGG, AGA, CTA, CCC, GGA, and ATA.

Preferably, the secondary structure of the mRNA transcribed from the polynucleotide encoding the polypeptide linker can form a higher order steric structure. More preferably, the higher order steric structure is a stem-loop structure. Even more preferably, the usage frequency of the amino acid encoded by the polynucleotide encoding the polypeptide linker is lower in the host cell transfected with the polynucleotide. The fusion protein is an antibody fragment. And even more preferably, the antibody fragment is scFv or sc(Fv)2.

It is particularly preferable that the polynucleotide encoding the polypeptide linker has the sequence set forth in SEQ ID NO: 6, 8, 10, 12, or 24.

Also referred to is a vector wherein the polynucleotide referred to herein is inserted so that the polynucleotide can be expressed in the host cell, as well as a host cell transformed by that vector. The host cell is a prokaryotic cell, more preferably *E. coli.*

The process of the invention is for producing a soluble antibody fragment. The process comprises preparing an antibody fragment gene with the sequence having been modified or designed so that the translation rate of a linker sequence joining the functional domains within the antibody fragment molecule is delayed, and culturing host cells transformed by the gene.

The process comprises preparing an antibody fragment gene whose sequence has been modified or designed so that the sequence of a linker contains rare codons in order to delay the translation rate of the linker, and culturing host cells transformed by the gene.

The process for producing a soluble antibody fragment may comprise preparing an antibody fragment gene whose sequence has been modified or designed such that the secondary structure of the encoded mRNA can form a higher order steric structure to delay the translation rate of the linker sequence, and culturing host cells transformed by the gene.

Also referred to is a nucleotide sequence of a linker to be used for joining the functional domains of the antibody fragment, wherein the nucleotide sequence is modified or designed so that the translation rate of the linker sequence is delayed when the gene encoding the antibody fragment is expressed in host cells transformed by the gene.

Also referred to is a nucleotide sequence of a linker to be used for joining the functional domains of the antibody fragment, wherein the nucleotide sequence is modified or designed so that the sequence of the linker comprises rare codons in order to delay the translation rate of the linker sequence when the gene encoding the antibody fragment is expressed in host cells transformed by the gene.

Also referred to is a nucleotide sequence of a linker to be used for joining the functional domains of the antibody fragment, wherein the nucleotide sequence is modified or designed such that the secondary structure of the mRNA can form a higher order steric structure to delay the translation rate of the linker sequence when the gene encoding the antibody fragment is expressed in host cells transformed by the gene.

Also referred to is a nucleotide sequence of a linker to be used for joining the functional domains of the antibody fragment, wherein the amino acid sequence of the linker comprises amino acids with low usage frequency in the host cells to delay the translation rate of the linker sequence when the gene encoding the antibody fragment is expressed in host cells transformed by the gene.

Also referred to is provides a gene encoding an antibody fragment comprising the aforementioned linker nucleotide sequence, a vector carrying the gene, and a host cell transformed by the vector.

Also referred to is a process for producing an antibody fragment. The process comprises culturing host cells transformed by a vector carrying a gene encoding an antibody fragment comprising the aforementioned linker nucleotide sequence, and purifying the antibody fragment from the culture medium.

By using the nucleotide sequence encoding the fusion protein linker, the fusion protein can be expressed in a soluble form in a host of a biological species different from that of the donor. In addition, by using the nucleotide sequence encoding the antibody fragment linker, the antibody fragment can be expressed in a soluble form in a host of a biological species different from that of the donor. In particular, an antibody fragment can be expressed in a soluble form when expressed in *E. coli.* Furthermore, by using the nucleotide sequence encoding the antibody fragment linker, the level of expression of the antibody fragment can be increased when the antibody fragment is produced in a host of a biological species different from that of the donor. In particular, the level of expression of the antibody fragment can be increased when the antibody fragment is expressed in *E. coli*. Additionally, by using the nucleotide sequence encoding the antibody fragment linker, scFv (single-chain Fv antibody fragment) and sc(Fv)2 can be expressed in a soluble form when the scFv and sc(Fv)2 are produced in a host of a biological species different from that of the donor. In particular, the scfv and sc(Fv)2 can be expressed in a soluble form in *E. coli.* Finally, by using the nucleotide sequence encoding the antibody fragment linker, the level of expression of the scFv and sc(Fv)2 can be increased when the scFv and sc(Fv)2 are produced in a host of a biological species different from that of the donor. In particular, the level of expression of the scFv and sc(Fv)2 can be increased when they are expressed in *E. coli.*

## BRIEF DESCRIPTION OF THE DRAWINGS

Figure 1 is a diagram showing the vector for panning;
Figure 2 is a diagram showing the vector for scFv expression;
Figure 3 is a graph showing bacterial cell growth after IPTG induction;
Figure 4 is a diagram showing analysis of three-dimensional structure by gel chromatography;
Figure 5 is a diagram showing analysis of three-dimensional structure by SDS-PAGE;
Figure 6 is a diagram showing the binding of scFv to an antigen-bound column;
Figure 7 is a diagram showing antigen recognition by biotinylated scFv;
Figure 8 is a diagram showing the productivity of the scFv soluble molecule;
Figure 9 is a diagram showing the ratio of the solubility of the scFv;
Figure 10 is a diagram showing the growth of host cells producing the scFv;
Figure 11 is a diagram showing the growth curve -of bacterial cells expressing the sc(Fv)2;

Figure 12 is a diagram showing the results of analysis of the sc(Fv)2 by SDS-PAGE; .
Figure 13 is a diagram showing the results of analysis of the sc(Fv)2 by Western blot;
Figure 14 is a diagram showing the time-course results of the intracellular insoluble fraction in bacterial cells expressing the sc(Fv)2;
Figure 15 is a diagram showing the time-course results of the intracellular soluble fraction in bacterial cells expressing the sc(Fv)2;
Figure 16 is a diagram showing the time-course results of the culture supernatant of the bacterial cells expressing the sc(Fv)2;
Figure 17 is a diagram showing a sensorgram obtained from Biacore analysis for the culture supernatant of the bacterial cells expressing the sc(Fv)2; and
Figure 18 is a magnified view of Figure 17.

## PREFERRED EMBODIMENTS OF THE INVENTION

### Fusion Protein

As used herein, the term "fusion protein" refers to an artificial protein or polypeptide produced by fusing two or more proteins or polypeptides by techniques based mainly on genetic engineering. The fusion protein of the invention is an antibody fragment that comprises variable domains joined by a polypeptide linker or linkers which consist of 5 to 50 amino acids. The terms "protein" and "polypeptide" are used herein interchangeably. Techniques based on genetic engineering (hereinafter referred to "genetic engineering techniques") are typically used in the process of producing fusion proteins, but the process of the invention is not limited to any specific techniques. The proteins constituting the fusion protein may be polypeptide domains that are regions necessary for exhibiting all or part of the functions of the full-length proteins. A chimeric protein is one type of fusion protein, and covers a broad range of proteins where the fused polypeptide domains of the proteins are derived from proteins of different species.

Each polypeptide domain may be a polypeptide domain originating in the same molecule. More specifically, if a naturally occurring full-length protein comprises the functional domains of <polypeptide domain A> + <polypeptide domain B> + <polypeptides domain C>, then any molecules wherein these functional domains are linked together are all included in the definition of fusion protein of the present invention, provided that the polypeptide domains are fused together by the polypeptide linker molecule described below and the protein is an antibody fragment. Such molecules may include, for example, a polypeptide molecule consisting of <polypeptide domain A> + <polypeptide domain B>, a polypeptide molecule consisting of <polypeptide domain A> + <polypeptide domain C>, or a polypeptide molecule consisting of <polypeptide domain A> + <polypeptide domain B> + <polypeptide domain C>.

Furthermore, as used herein, the term "polypeptide" covers a broad definition of polypeptide, including peptides with a chain length shorter than 50 amino acids. In accordance with this definition, the number of amino acid residues constituting the polypeptide by peptide bonds is not limited, and may include polypeptides ranging from 1 to 3000 amino acids. Preferably, a polypeptide comprises 1 to 1500 amino acids, more preferably 1 to 1000 amino acids, even more preferably 1 to 500 amino acids, and most preferably 1 to 300 amino acids.

The genetic engineering techniques for preparing the "fusion protein" are briefly listed below. A polynucleotide is artificially prepared with a nucleotide sequence wherein a polynucleotide sequence encoding a polypeptide domain of a certain protein is fused in-frame with a polynucleotide sequence encoding a polypeptide domain of a different protein, and additionally it has the start codon ATG in-frame on the 5' end, and the stop codon TAA, TGA, or TAG in-frame on the 3' end. Thus, recombinant DNA encoding the target fusion protein in a single reading frame can be constructed. For such genetic engineering techniques, a person skilled in the art may refer to the procedures described in "Molecular Cloning, A Laboratory Manual," Cold Spring Harbor Laboratory Press, NY, Vol. 1, 2, 3 (1989)), etc., and produce the fusion protein with an ordinary level of creativity by a person skilled in the art.

When a gene encoding a fusion protein originating in a eukaryotic organism and having been prepared with the genetic engineering techniques is expressed in prokaryotic cells such as *E. coli* and the like, sometimes the protein will not be expressed, or even if the protein is expressed, an active form of the protein cannot be obtained because of the formation of inclusion bodies. In order to solve these problems, the present invention provides a production process wherein the formation of inclusion bodies is suppressed, and the fusion protein can be produced more in a soluble form.

As used herein, the term "soluble" refers to a molecular form which is secreted into the cytoplasm or periplasm of the recipient cell, or the supernatant of the culture medium without the formation of insoluble inclusion bodies in the cytoplasm of the recipient cell. Determination of the presence of soluble and insoluble forms of the fusion protein is described in the examples below, and can be carried out according to the method of Knappik et al. (Protein Eng., 8(1), 81-9, 1995). Preferably the method specifically described in the examples and the like may be used for analyzing the content of soluble and insoluble fusion protein molecules to verify the effect of the present invention.

In addition, a linker (also called a spacer) sequence is used to fuse the polypeptide domains originating in different proteins to produce the fusion protein. The amino acid sequence constituting the linker is not particularly limited, but is preferably designed so that the function of each polypeptide domain in the fusion protein is retained. For the antibody fragment produced as a fusion protein, the sequence GGGGSGGGGSGGGGS(SEQ ID NO: 13) (hereinafter, called (G4S)3) is generally used as a linker. The linker is not limited to the above sequence, provided the function of each polypeptide domain in the fusion protein is retained. The length of the amino acid sequence of the linker of the present invention is is 5 to 50 amino acids, preferably 13 to 30 amino acids, and more preferably 15 to 30 amino acids. The linker is explained in greater detail herein below. The polynucleotide sequence encoding the linker has one or more rare codons, where a rare codon is one which has a tRNA ratio of 1.2% or less of the total tRNA in a prokaryotic host cell.

The effect of the present invention can be obtained by modifying the polynucleotide sequence encoding a polypeptide that functions as a spacer joining each functional domain according to the present invention. Namely, the polynucleotide sequence is modified to a "sequence such that the translation rate of the spacer is delayed when the mRNA generated by transcription of the nucleotide sequence is translated." Delay of the translation rate refers to a situation where the translation rate of the mRNA region encoding the polypeptide linker is slower than the translation rate of the mRNA region encoding the polypeptide domain immediately upstream of the linker.

Also referred to is a process for producing a naturally occurring protein comprising the following steps:

(1) determining the polypeptide domains present in the molecule of the naturally occurring protein;
(2) determining the polynucleotide sequence encoding the polypeptide sequence(s) lying between each polypeptide domain;
(3) producing a polynucleotide having the sequence modified or designed so that the translation rate of the polynucleotide sequence is delayed; and
(4) culturing a host transformed by a vector having the polynucleotide inserted into the vector to allow for expression.

Polypeptide domains present in naturally occurring protein molecules are recorded in a publicly shared database such as PROSITE, PROFILE, ProDom, Pfam, etc. A publicly shared motif search program such as Pfam (http://pfam.wustl.edu/hmmsearch.shtml) may be used to determined what kinds of polypeptide domains are present in the sequence of a protein of interest, and to which fragments the polypeptide domains correspond in the amino acid sequences of the polypeptide of the protein of interest.

Also referred to is a process for producing a protein by modifying the codons in a polypeptide functional domain of a naturally occurring protein molecule to a "sequence such that the translation rate is delayed when the mRNA generated by transcription of the nucleotide sequence is translated." In this case it is preferable that the amino acid residue encoded by the codon involved in modification is essentially the same as the amino acid residue before modification, but is not limited. The term "essentially the same" refers to interrelationships among amino acids wherein the properties of the side chains of the amino acid residues are conserved. For example, properties of amino acid side chains include hydrophobic amino acids (A, I, L, M, F, P, W, Y, V), hydrophilic amino acids (R, D, N, C, E, Q, G, H, K, S, T), amino acids having an aliphatic side chain (G, A, V, L, I, P), amino acids having a side chain containing a hydroxyl group (S, T, Y), amino acids having a side chain containing a sulfur atom (C, M), amino acids having a side chain containing carboxylic acids and amides (D, N, E, Q), amino acids having.a side chain containing a base (R, K, H), and amino acids having a side chain containing an aromatic group (H, F, Y, W) (The letters in parentheses represent the single letter abbreviations of amino acids). The interrelationship of amino acids in each group listed above is considered to be "essentially the same."

Antibody Fragment

The fusion protein of the present invention is an antibody fragment where the antibody fragment comprises variable domains joined by a polypeptide linker or linkers which consist of 5 to 50 amino acids.

The full-length antibody molecules that form the basis of the antibody fragment molecule can be classified into 5 general classes of IgA, IgD, IgE, IgG, and IgM based on the characteristics of their molecular structures. The basic structure is shared by all classes, and consists of heavy chains (H-chains) comprising polypeptide chains with a molecular weight of 50,000 to 70,000 and a light chains (L-chains) comprising polypeptide chains with a molecular weight of 20,000 to 30,000. Two homologous H-chain molecules and two homologous L-chain molecules are linked by disulfide bonds and non-covalent bonds. The N-terminal polypeptide domains of the H-chains and L-chains do not have mutually identical amino acid sequences even in antibodies of the same class, and so they are called variable regions (V-regions). The variable region of the heavy chain is called the heavy-chain variable region (VH) and the variable region of the light chain is called the light-chain variable region (VL). The structures and amino acid sequences of polypeptide domains other than the variable regions are constant in each class (or subclass in the case of subclasses) and are called constant regions (C regions). The constant region of the heavy chain is called the heavy-chain constant region (CH), and the constant region of the light chain is called the light-chain constant region (CL). The antigen binding site constitutes a VH and VL, and the binding specificity toward the antigen is determined by the amino acid sequence of the variable region. In general the VH contributes largely to the specificity and affinity toward the antigen than VL (Nature, 341, 544-6, 1989). It has been shown that, however, the VH polypeptide alone is inadequate for use as a molecule that retains sufficient binding activity toward the antigen, because VH has low solubility due to the presence of hydrophobic residues at the interface to VL, and the VH region itself has low affinity toward the antigen.

Skerra et al. discovered that the Fv molecule, a heterodimer assemblage of the VH polypeptide and the VL polypeptide, is secreted into the periplasm of *E. coli* by expressing the genes encoding the VH polypeptide and VL polypeptide positioned downstream from a signal peptide domain. The Fv molecule is a heterodimer constituted by VH and VL in a one-to-one assembly. Because the mutual interaction is weak between the VH polypeptide and VL polypeptide with the dissociation constant being in the range of $10^{-5}$ to $10^{-8}$ M, the Fv molecule has the disadvantage of an unstable molecular structure of a heterodimer. Then scFv (single-chain antibody fragment) has been designed and produced by genetic engineering techniques (Proc. Natl. Acad. Sci. USA., 85(16), 5879-83, 1988), and the problem of instability in the molecular structure as a heterodimer was solved. scFv is a single-chain antibody wherein the VH polypeptide and VL polypeptide are joined by a polypeptide linker.

However, when the scFv is expressed in prokaryotic cells such as *E. coli* as host cells, scFv may aggregate together to form inclusion bodies, or suitable folding of the polypeptide chain does not occur. Therefore efficient production process of scFv has been demanded. Meanwhile, it was also known that additional functions such as a bispecificity could be imparted by making the scFv into a dimer (Diabody). A method for efficient production of scFv dimer (Diabody) is also demanded. The present invention provides a process for producing scFv molecules and scFv dimer diabodies in a soluble form without the formation of inclusion bodies.

Furthermore, the scFv of the present invention may encompass a protein comprising scFv. Examples of a protein containing scFv include a fusion protein comprising a domain polypeptide of another protein fused to scFv (hereinafter called an scFv fusion protein) and sc(Fv)2 wherein two scFv molecules are joined together. The domain polypeptide of another protein may include, for example, an antibody Fc region polypeptide. In this regard, the antibody fragment of the present invention encompasses scFv as well as an scFv fusion protein, sc(Fv)2, and the like. A polynucleotide encoding such antibody fragments is also referred to.

The antibody fragment produced is a fusion protein wherein variable domains are joined via the polypeptide linker described herein. The variable regions may be a full-length variable region, or a partial peptide of a variable region. Also included is a polypeptide comprising another polypeptide added to the variable region. Preferably, the antibody fragment maintains the binding activity toward the antigen. The antigen to be recognized by the antibody fragment is not particularly limited, and the invention is applicable to any antigens.

Additionally, the scFv may be a diabody designed as a bispecific antibody that recognizes two different antigens when formed into a dimer. A diabody is a dimer consisting of two polypeptide chains, and typically each of the polypeptide chains comprises VL polypeptide and the VH polypeptide connected by a short polypeptide linker, for example, of about 5 amino acid residues, such that the VL and VH polypeptides in the same molecule cannot bind to each other (Proc. Natl. Acad. Sci. USA., 90, 6444-8, 1993). The VL polypeptide and VH polypeptide located on the same polypeptide chain cannot form an single chain variable region fragment because the linker lying between them is too short, and they assemble into a dimer to form a diabody having two antigen binding sites. The bispecific antibody can be a bispecific antibody that recognizes different antigens, or it can be a bispecific antibody that recognizes different epitopes on the same antigen. Furthermore, it can be a bispecific antibody wherein one antigen binding site recognizes a protein, and the other antigen binding site recognizes a cytotoxic substance such as a chemotherapy drug, a toxin of cellular origin, and the like. The present invention may be used to produce sc(FV)2 that is designed to be a monomeric bispecific antibody.

Typically, sc(Fv)2 is an antibody wherein four variable regions consisting of two VL peptides and two VH peptides are joined by linkers into a single chain (Hudson et al., J. Immunol. Methods., 231, 177-189, 1999). sc(P'v)2 can be produced by the same method as scFv, but the order of two VHs and two VLs is not particularly limited. It can be constructed in a plurality of modes as shown below.

(N-terminus) [VH] linker [VL] linker [VH] linker [VL] (C-terminus)
(N-terminus) [VL] linker [VH] linker [VH] linker [VL] (C-terminus)
(N-terminus) [VH] linker [VL] linker [VL] linker [VH] (C-terminus)
(N-terminus) [VH] linker [VH] linker [VL] linker [VL] (C-terminus)
(N-terminus) [VL] linker [VL] linker [VH] linker [VH] (C-terminus)
(N-terminus) [VL] linker [VH] linker [VL] linker [VH] (C-terminus)

In the sc(Fv)2 molecules described above, a linker encoded by the polynucleotide described herein can be used at any site or at a plurality of locations of the linker connecting each functional polypeptide domain.

In the case of sc(Fv)2 designed to be a monomeric bispecific antibody, there are structural isomers of the single-chain diabody type and the bivalent scFv type with respect to the positional relationship in the molecule of the VL and VH that assemble to form an antigen binding sites. In the present invention, if the functional polypeptide domains in the sc(Fv) 2 molecule are arranged in the following order from the N-terminus: [V-region 1] linker [V-region 2] linker [V-region 3] linker [V-region 4] linker, the term "single-chain diabody type" refers to an sc(Fv)2 molecule having a structure wherein V-region 1 and V-region 4, and V-region 2 and V-region 3 each assembles to form a single antigen binding site. On the other hand, when the functional polypeptide domains in the sc(Fv)2 molecule are arranged in the following order from the N-terminus: [V-region 1] linker [V-region 2] linker [V-region 3] linker [V-region 4] linker, the term "bivalent scFv type" refers to an sc(Fv)2 molecule having a structure wherein V-region 1 and V-region 2, and V-region 3 and V-region 4 each assemble to form a single antigen binding site.

A linker encoded by the polynucleotide described herein can be used at any site or at a plurality of locations in the linker.connecting functional polypeptide domains in either structural isomer of the sc(Fv)2 molecule described above.

Also referred to is a polynucleotide encoding an antibody fragment designed in the above manner. When the polynucleotide described herein is expressed in prokaryotic cells such as *E. coli* and the like, it is possible to suppress the formation and accumulation of inclusion bodies and allow for expression of the polypeptide in a soluble form by controlling the translation rate at the translation stage of expression.

Polypeptide Linker Used to Prepare scFv and Other Molecules

As noted above, the problem of unstable molecular structure of the Fv molecule in a heterodimer has been solved by the establishment of scFV preparation techniques. In general, an amino acid sequence that is widely used as a polypeptide linker is (G4S)3 having the sequence GGGGSGGGGSGGGGS (SEQ ID NO: 13). It contains a large number of glycine residues such that it will not have a constrained secondary structure, and it has serine residues inserted to preserve hydrophilicity. This type of polypeptide linker is generally called a flexible linker. Because the formation of inclusion bodies may not prevented even when the above polypeptide sequence is used, multiple attempts have been made to design a polypeptide linker that has another amino acid sequence and that can also avoid inclusion body formation (J. Immunol. Methods, 205(1), 43-54, 1997 and Prot. Eng., 11(5), 405-410, 1998).

The present invention employs a polypeptide linker that can be used to join the fusion protein in-frame, and a polynucleotide encoding the polypeptide linker so that the reading frame of each domain of a protein or polypeptide fused via the linker sequence is not changed. The fusion protein of the present invention is an antibody fragment such as scFv fusion protein and sc(Fv)2 in addition to scFv.

Specific examples of the amino acid sequence of the linker are described in detail in the examples below. Preferred linkers have the following amino acid sequence:

WVWSSRGQRSFRPSGRTVPL (SEQ ID NO: 5) (hereinafter, called linker No. 10);
KWLWTTRVRDRGHTSTMWS (SEQ ID NO: 7) (hereinafter, called linker No. 12);
ADGHCHLKNFPLKPPPYFSV (SEQ ID NO: 9) (hereinafter, called linker No. 14);
LLKKLLKKLLKKLLKK (SEQ ID NO: 11) (hereinafter, called (LLKK)4); and .
GGGGSGGGGSGGGGS (SEQ ID NO: 13).

Polynucleotide Encoding Polypeptide Linker Used to Prepare scFv and Other Molecules

The nucleotide sequence encoding,the polypeptide linker is designed so that when mRNA produced by transcription of that nucleotide sequence is translated, the translation rate of the linker is delayed. A polynucleotide having any nucleotide sequence may be used in the invention provided that the domains of the protein or polypeptide fused by the linker can be joined in-frame so that the reading frame does not change. As a preferred example, a linker having the following nucleotide sequence may be used, which is described in detail in the examples below:

TGGGTTTGGAGTTCGCGGGGGCAGAGGTCTTTTCGGCCTTCGGGGCGGACGGTGCCGCTT (SEQ ID NO: 6);
AAGGUUGUUCUUUGGACUACGCGUGUUAGGGAUAGGGGUCAUACGUCGACGAUGUGGAGU (SEQ ID NO: 8);
GCGGATGGGCATTGTCATCTGAAGAATTTTCCTTTGAAGCCTCCGCCTTATTTTTCGGTT (SEQ ID NO: 10);
CTACTAAAAAAACTACTAAAAAAACTACTAAAAAAACTACTAAAAAAA (SEQ ID NO: 12); and
GGTGGAGGCGGTTCCGGCGGAGGTGGCTCCGGCGGTGGCGGATCC (SEQ ID NO: 14)

The present invention enables a target antibody fragment to be expressed in soluble form at a high level of expression in *E. coli* by modifying the genetic sequence of the polypeptide linker of the antibody fragment and thereby delaying the translation rate of the antibody fragment locally at the linker. Delaying the translation at the polypeptide linker is thought to greatly affect post-translational folding of both N- and C-terminal domains (in the case of an scFv, the VH and VL polypeptide domains). Delaying translation at the linker as described above means that the folding of the N-terminal polypeptide domain will precede translation and folding of the C-terminal polypeptide domain. It is believed that by delaying translation at the linker, folding of the N-terminal domain, which has already been translated, will be little interfered by the C-terminal polypeptide domain, which is still be being translated and has not folded yet. It is also believed that folding of the C-terminal polypeptide domain will proceed more efficiently under conditions where the N-terminal polypeptide domain has already folded to a certain extent compared to the case in which the folding occurs under conditions wherein both polypeptide domains are not yet folded. Therefore, if the nucleotide sequence of the polypeptide linker of the antibody fragment is designed such that the translation rate is temporarily delayed at the polypeptide linker, solubility and productivity of the antibody fragment may be improved. In the process for producing the antibody fragment, the VH and VL polypeptide domains constituting the antibody fragment can be located at either end, i.e., either on the N-terminal side or C-terminal side, of.the polypeptide linker with a sufficient intermolecular distance to allow for assembling together when expressed as a single polypeptide chain.

As used herein, the phrase "sequence such that the translation rate is delayed when the mRNA generated by transcription of the nucleotide sequence is translated" means a sequence having any characteristics and structure provided that it will delay the translation rate in the recipient cells. Methods for quantifying the translation rate are known to those skilled in the art. Translation rate may be measured by referring to the literature accompanying a commercial measurement kit (for example, *E. coli* S30 Extract System by Promega, etc.). In addition to the above methods carried out in a test tube, a method for measuring the translation rate *in vivo* is also known as described in Mol. Immunol. 40, 717-22, 2004.

The phrase "sequence such the translation rate is delayed that when the mRNA generated by transcription of the nucleotide sequence is translated" may be, for example, a sequence containing a rare codons. The linker(s) employed in the invention comprise one or more rare codons. The term "rare codon" generally refers to a codon having a low usage frequency in the recipient cells. However, it is known that codon usage frequency and the corresponding tRNA concentration in an organism do not always match perfectly. A rare codon is defined herein as that the tRNA ratio of a certain codon is 1.2% or less of the total tRNA. It is known that the translation rate varies in a concentration dependent manner With the abundance of ribosomes and with the tRNA that is complementary to the triplet corresponding to each mRNA codon, but the codon usage frequency does not match perfectly to the corresponding tRNA concentration (Microbiol. Rev., 54(2), 198-210, 1990). If a codon recognized by a low concentration of tRNA is to be called a rare codon in *E. coli,* then the term refers to GCC (Ala, 0.95%), CGG (Arg, 0.99%), AGG (Arg, 0.65%), CAA (Gln, 1.18%), CAC (His, 0.99%), CAT (His, 0.99%), CTA (Leu, 1.03%), CCC (Pro, 1.11%), CCA (Pro, 0.90%) and TCC (Ser, 1.18%) (notations in parentheses refer to the amino acid corresponding to each codon and the frequency in consideration of tRNA concentration).

Therefore, the term "rare codon" as used herein includes rare codon as used in the normal sense, as well as a codon recognized by tRNA at a low concentration in the recipient cells. In the case of *E. coli,* the term rare codon used in the normal sense is one calculated from the frequency of the occurrence of codons found on the genome, as reported in several publicly known documents (for example, http://www.kazusa.or.jp/codon/cgi-bin/showcodon.cgi?species=Escherichia+coli+%5Bgbbct%5D). Examples of such codons include AGG (Arg, 0.26%), AGA (Arg, 0.46%), DATA (Ile, 0.85%), CTA (Leu, 0.46%), CCC (Pro, 0.56%), GGA (Gly, (1.08%), and CGG (Arg, 0.66%) (notations in parentheses refer to the amino acid corresponding to each codon and the frequency of occurrence of cordons on the genome). A codon falling within either definition can be used in the present invention.

In the present invention the number of rare codons to be introduced is not particularly limited and any number of rare codons can be used in designing the polypeptide linker sequence. For example, if the polypeptide linker consists of 20 amino acids in length, the number of rare codons to be introduced can range from 1 to 20, preferably 1 to 15, and even more preferably 1 to 10. Even more preferable is a number ranging from 2 to 10, and a number from 2 to 5 is especially preferred.

Even if the nucleotide sequence encoding the polypeptide linker already contains a rare codon, the level of expression of the soluble form of the antibody fragment can be further improved by increasing the number of additional rare codons in that nucleotide sequence.

A polynucleotide having any nucleotide sequence can be used as the polypeptide linker. For example, a "sequence such that the translation rate is delayed in the recipient cells when the mRNA generated by transcription of that nucleotide sequence is translated" containing a rare codon can be newly designed. In addition, as shown in the examples below, a certain codon in a known polypeptide linker can be modified into a "sequence such that the translation rate is delayed in the recipient cells

Another example of a "sequence such that the translation rate is delayed in the recipient cells when the mRNA generated by transcription of that nucleotide sequence is translated," other than the sequence described above is a sequence wherein the secondary structure of .the mRNA can form a higher-order steric structure. Such sequence forms a secondary structure stable enough to temporarily prevent ribosome from moving along the mRNA when the polypeptide is translated from the mRNA. A preferred example of the secondary structure is a sequence that forms a thermodynamically stable, regional double strand in mRNA, a so-called stem-loop structure.

Preferred examples may include a sequence that forms a stable regional secondary structure such that a plurality of nucleotides in the polynucleotide sequence encoding the polypeptide linker of the transcribed mRNA will form hydrogen bonds (A-U, G-C or G-U). In that sequence a thermodynamically stable, "stem-loop" secondary structure formed through a series of contiguous hydrogen bond pairs will be formed.

Such a stem-loop secondary structure, along with the free energy values of that structure, can be predicted, for example, by RNAfold (http://rna.tbi.univie.ac.at/cgi-bin/RNAfold.cgi), and by a genetic analysis program such as http://www.nanobiophys-sakura.net/HyFol/HyFoll.html).

In addition, the "sequence such that the translation rate is delayed in the recipient cells when the mRNA generated by transcription of that nucleotide sequence is translated" may include a sequence encoding an amino acid with a low usage frequency in the host cells into which the polypeptide is introduced. The term "an amino acid with a low usage frequency" means the top 10 amino acids with the lowest frequency, preferably 8 amino acids, more preferably 5 amino acids, still more preferably 1 amino acid with the lowest frequency, when the amino acids are aligned in order of usage frequency in a specific host cell.

In a preferred embodiment, an amino acid with a low usage frequency in the host cells into which the fusion protein is introduced may be identified by analyzing the amino acids constituting a polypeptide sequence intended to function in the host cells based on genomic data of the host cells, and then calculating the usage frequency of the amino acid. When the host cells are bacteria, the amino acid sequences of hypothetical reading frames are recorded in a public database (for example, TIGR: http://cmr.tigr.org/tigr-scripts/CMR/shared/Genomes.cgi?bacteria_only=1), and the frequency of occurrence of amino acids in such a sequence can be calculated by using a genetic analysis program (e.g., Genetyx (Genetyx Corporation, Japan). An example of analytical results that were calculated based on genomic data can be found in Protein Science 14, 617-25, 2005.

If the entire genomic data of the host cell is not published, the usage frequency may be analyzed by the genetic analysis program using a plurality of polypeptides that originated in the host cells and whose sequences have been recorded in a database.

When E. *coli* are the host cells, examples of such amino acids include tryptophan, cysteine, histidine, methionine, tyrosine, glutamine, phenylalanine, etc.

The number of amino acid residues that are to be introduced and have a low usage frequency in the host cells is not particularly limited in the design of the polypeptide linker sequence and any number of residues may be used. For example, if the polypeptide linker consists of 20 amino acids in length, the number of those amino acid residues to be introduced can range from 1 to 20, preferably 1 to 15, and even more preferably 1 to 10. Even more preferable is a number ranging from 2 to 10, and a number from 2 to 5 is especially preferred.

The polynucleotide encoding the linker can be produced by methods known to those skilled in the art. Synthetic DNA having a specific sequence can be produced by solid phase synthesis using semi-automated equipment (for example, Models 392/394 from PE Applied Biosystems). After the polynucleotide encoding the polypeptide linker has been synthesized by chemical synthesis together with its complementary strand using the above method, it can be thermally denatured (e.g., 10 min at 95°C), annealed, and cloned into a vector as double-stranded DNA, or it can be amplified by PCR. The PCR method is preferably used. In particular, fusion PCR, a modified PCR method may preferably used for producing a fusion DNA with the polynucleotide encoding the polypeptide domains to be joined by the linker (Biotechniques, 12(6), 864-9, 1992).

. To introduce a rare codon of the recipient cells, a nucleotide sequence that forms a stem-loop structure, or a nucleotide sequence encoding an amino acid residue with a low usage frequency in the host cells into the polypeptide linker, a nucleotide sequence having a rare codon, a nucleotide sequence that forms a stem-loop structure, or a nucleotide sequence encoding an amino acid residue with a low usage frequency in the host cells may be synthesized by the above synthetic procedure. Also, a mutation may be introduced into an existing nucleotide sequence, as shown in the Examples below. A mutation may be introduced into the polynucleotide encoding the polypeptide linker according to a method of introducing a mutation that is well known in the art, for example, the site-directed mutation (Gene, 152, 271-5, 1995; Methods in Enzymol., 100, 468-500, 1983; Nucleic Acids Res., 12, 9441-56, 1984; Methods in Enzymol. 154, 350-67, 1987; Proc. Natl. Acad. Sci. USA., 82, 488-492, 1985; and Methods in Enzymol. 85, 2763-6, 1988).

The polypeptide linker itself may have some function in addition to joining the polypeptide domains in-frame. For example, if the linker is designed to contain a protease cleavage domain, the fusion protein may be produced and then digested with a protease to obtain polypeptide domains constituting the fusion protein separately. In addition, if the linker is designed to contain an antigenic epitope sequence, the fusion protein may be produced and then purified using an antibody that recognizes that epitope (Biochem. Biophys. Res. Commun., 192(2), 720-7, 1993). Furthermore, it will be possible to detect the fusion protein by using an antibody that recognizes that epitope. The number of functional domains that can be inserted in such a linker is not limited to one, but two or more types of domains can be inserted into the linker.

DNA Encoding scFV and Vector Carrying the DNA

DNA encoding scFv can be obtained by using all or part of. the DNA sequence encoding the VH and VL polypeptides as a template and amplifying them by PCR using a primer pair defining both ends, then further amplifying by PCR using a primer pair designed such that both ends of the DNA encoding the polypeptide linker are joined to the respective heavy chain and light chain. Once the DNA encoding scFV is produced, an expression vector containing the DNA and a host transformed by the expression vector can be obtained by conventional methods. The scFv can be obtained using the host by conventional methods.

The sequence of the VH polypeptide and VL polypeptide in the scFv of the present invention may be obtained from already known sequences, or may be newly obtained using methods known to those skilled in the art. The ..present invention also encompasses DNA encoding the polypeptide linker of the present invention or the scFv polypeptide of the present invention. The DNA sequence produced as above is inserted into a vector that can drive expression in a suitable host such as a eukaryotic cell at downstream of a transcription element such as a promoter sequence and/or enhancer sequence and upstream of a poly-A signal sequence. The antibody fragment encoded by the DNA sequence can be expressed in cells transformed by the vector. If a Kozak sequence (for example, CCACC, etc.), which is a eukaryote ribosome binding sequence, is inserted between the transcription element and the start codon, translation efficiency can be increased, and the level of expression of the target antibody fragment can be increased. If secretion of the antibody fragment into the culture medium is desired, the vector can be designed so that a signal sequence serving as a protein secretion domain may be inserted downstream of the start codon.

Examples of vectors that can be used to produce the fusion protein include: expression vectors originating in mammals (e.g., pcDNA3 (Invitrogen) and pEGF-BOS (Nuc. Acids Res., 18(17), 5322, 1990), pEF, and pCDM8)): expression vectors originating in insect cells (e.g., "Bac-to-BAC baculovirus expression system" (GIBCO BRL), and pBacPAK8 (Clontech)); expression vectors originating in plants. (e.g., pSB1 and pSB2 (both in J. Gen. Microbiol., 130(10), 2527-34, 1984)); expression vectors originating in animal ' viruses (e.g., pHSV (Proc. Natl. Acad. Sci.. USA., 84(5), 1177-81, 1987), pMV-7 (DNA, 7(3), 219-25, 1988), and pAdex1 cw (Proc. Natl. Acad. Sci. USA., 93(3), 1320-4, 1996)); expression vectors originating in retroviruses (e.g., pZipNeo (Cell, 37, 1053-1062, 1984)); and expression vectors originating in yeast (e.g., "Pichia Expression Kit" (Invitrogen), pNV11 (Nature 357, 700-702, 1992) and pESP-3 (Stratagene).

Preferably, the expression vector will have a gene for selecting cells transformed by the vector (for example, a drug resistance gene that enables selection by a drug (such a neomycin, G418, etc.)). More specifically, when a group of cells subjected to transformation are cultured in a culture medium containing the drug at a concentration such that the host cells used for transformation normally cannot survive, but only cells transformed by the vector containing the drug resistance gene will be able to grow in the medium containing the drug through expression of the drug resistance gene present on the vector. Examples of vectors having such a property include pMAM and pDR2 (Clontech), and pBK-RSV, pBK-CMV, pOPRSV, and pOP13 (Stratagene).

When the fusion protein is expressed in mammalian cells such as CHO cells, COS cells, NIH3T3 cells, and the like, examples of preferable promoters may include: SV40 promoter (Nature, 277, 108, 1979), MMLV-LTR promoter (Oncogene, 7(10), 2081-3, 1992), EF1$\alpha$ promoter (Nuc. Acids. Res., 18, 5322, 1990), and CMV promoter (US Patent No. 5168062).

When using *E. coli* as the host, a ribosome binding sequence (RBS) is inserted downstream of the promoter sequence, and the DNA sequence prepared in the above manner is inserted downstream of the RBS sequence, so that the fusion protein encoded by the DNA sequence is expressed in *E. coli* transformed by a vector without using an enhancer sequence and poly-A signal. If expression of the fusion protein in the periplasm is desired, the vector can be designed so that a signal sequence operating as a protein secretion domain is inserted downstream of the start codon.

When using *E. coli* as the host cells transfected with the vector, the vector will have a replication origin ("ori") allowing for amplification of the vector and preparation of the protein in large quantities in *E. coli* (for example, strains JM109, DH5$\alpha$, HB101 (Toyobo) and XL1Blue (Stratagene)). The vector will also have a gene for selecting the transformed *E. coli* (for example, a drug resistance gene that enables selection by the drug (e.g., ampicillin, tetracycline, kanamycin, or chloramphenicol). Examples of such a vector include M13, pUC19, and pBR322 (Takara Shuzo), pBluescript and pCR-Script (Stratagene), and the like. In addition to the above mentioned vector, pGEM-T (Promega), pDIRECT (Clontech), pT7 (Novagen) and the like may be used for subcloning and excision of cDNA. For the purpose of producing the scFv, an expression vector is especially useful. For the purpose of expressing the protein in *E. coli*, for example, in *E. coli* strain such as JM109, DH5$\alpha$, HB101 and XL1Blue, the vector will have a characteristic that it can be amplified in *E. coli,* and also comprise a promoter that enables more efficient expression in *E. coli,* e.g., lacZ promoter (Nature, 341, 544-6, 1989, and FASEB J., 6, 2422-7, 1992), araB promoter (Science, 240, 1041-3, 1988) or T7 promoter (Proc. Natl. Acad. Sci. USA., 72(3), 784-8, 1975) and the like. Examples of such a vector include pGEX-5X-1 (Amersham Pharmacia), "QIAexpress system" (QIAGEN), pEGFP (Clontech) and pET( Novagen). In this case, a preferred host is BL21, which expresses T7 RNA polymerase.

A signal sequence for driving polypeptide secretion can also be included in the vector. The pelB signal sequence (J. Bacteriol., 169, 4379, 1987) can be used as a signal sequence for protein secretion into periplasm of *E. coli.* The vector may be introduced into the host cells by, for example, the calcium chloride technique or electroporation technique.

Transformant with Vector Carrying DNA Encoding scFv Also referred to are host cells comprising the vector described herein. The host cells to be transformed by the vector are not particularly limited, but any prokaryotic cells can be used. *E. coli* is a particularly preferable host to be used.

The vector may be introduced into the host cells by known methods such as the calcium phosphate technique, DEAE dextran technique, techniques based on the cationic liposome DOTAP (Roche Diagnostics), electroporation technique, lipofection, a Biolistic technique and the like.

Various bacterial production systems are known. Bacterial strains include; *E. coli* strains, such as JM109, DH5$\alpha$, HB101, and *Bacillus* subtilis strains Marburg 168 and BD170, and *Bacillus licheniformis.*

The antibody fragment can be produced by transforming these cells with the target DNA and culturing the transformed cells in vitro.

The antibody fragment expressed and produced as described above can be purified by using known methods conventionally used for protein purification either alone or in suitable combinations. For example, the antibody fragment can be isolated and purified by suitable selection and combination of an affinity chromatography column, ion chromatography, hydrophobic chromatography column, gel chromatography column, filtration, ultrafiltration, salting out, dialysis, and the like (see, for example, Antibodies: A Laboratory Manual. Ed Harlow, David Lane, Cold Spring Harbor Laboratory, 1988).

Pharmaceutical Composition

Also referred to is a pharmaceutical composition comprising the antibody fragment as an active ingredient. The pharmaceutical composition can be formulated by conventional methods (for example, Remington's Pharmaceutical Science, latest edition, Mark. Publishing Company, Easton, U.S.A.) It can also comprise a pharmaceutically permissible vehicle and additives, for example, but are not limited, a surfactant, bulking agent, coloring agent, flavor, preservative, stabilizer, buffer, suspending agent, isotonic agent, binder, disintegrant, lubricant, flow promoter, flavor enhancer, and other conventionally used excipients. Specific examples include light silicic acid anhydride, lactose, crystalline cellulose, mannitol, starch, carmellose calcium, carmellose sodium, hydroxypropyl cellulose, hydroxypropyl methylcellulose, polyvinyl acetal diethyl aminoacetate, polyvinyl pyrrolidone, gelatin, medium-chain fatty acid triglycerides, polyoxyethylene-hydrogenated palm oil 60, sucrose, carboxymethyl cellulose, corn starch, inorganic salts, and the like.

The pharmaceutical composition may be administrated either orally or parenterally. A parenteral method of administration is particularly preferred and may include injection, transnasal administration, transpulmonary administration, transcutaneous administration and the like. As an example of administration by injection, the pharmaceutical composition can be administered systemically or locally by intravenous injection, intramuscular injection, intraperitoneal injection, subcutaneous injection, and the like. A suitable method of administration can be selected depending on the age and condition of the patient. For example, a dosing amount in the range of 0.0001 mg to 1000 mg/kg of body weight per dose can be selected. Alternatively, a dosing amount in the range from 0.001 mg to 100,000 mg/body per patient can be selected. However, the pharmaceutical composition is by no means limited by these dosing amounts.

EXAMPLES

The present invention is explained in greater detail by the following examples, but is by no means limited by the examples.

Example 1: Anti-bisphenol A antibody scFv

<Construction of scFv library having random polypeptide linker>

To investigate the effect of the polypeptide linker sequence on scFv productivity, a phage library of scFv having a polypeptide linker consisting of a random amino acid sequence of 20 amino acids in length was prepared and selected by panning using bisphenol A as the antigen. More specifically, the VH gene and VL gene were amplified by PCR from the gene of an scFv having a flexible linker. An antibody gene having the sequence of AB097940 was used as the PCR template (K. Nishi, M. Takai, K. Morimune and H. Ohkawa, Molecular and Immunochemical Characteristics of Monoclonal and Recombinant Antibodies Specific to Bisphenol A. Bioscience, Biotechnology and Biochemistry, 67(6), 1358-1367 (2003)). An oligonucleotide was synthesized having the 17 residues of the 3' terminal of the VH gene, a random linker gene (NNK) 20, and the 17 residues of the 5' terminal of the VL gene were synthesized (5'-CAGTCACCGTCTCCTCA(NNK)20GACATTGTGCTGACACA-3', SEQ ID NO: 19). Using the VH and VL genes and this oligonucleotide, overlapping PCR was performed, and random polypeptide linker genes were inserted between the VH gene and the VL gene. The plasmid shown in Figure 1 was used as the vector for panning.

The following primers were prepared:

NcoI-cc-VH sense (Tm 68°C) (SEQ ID NO: 20)
5'-CTCCCATGGCCGATGTACAGCTTCAGGAGTCAGGACCTGCC-3'
BIS A VH antisense (Tm 61°C) (SEQ ID NO: 22)
5'-TGAGGAGACGGTGACTGAGGTTCCTTGACC-3'
BIS A VL sense (Tm 59°C) (SEQ ID NO: 23)
5'-GACATTGTGCTGACACAGTCTCCTGCTTCC-3'
VL+NotI antisense (Tm 64°C) (SEQ ID NO: 21)
5'-ATATATGCGGCCGCCCGTTTGATTTCCAGCTTGGTGC-3'.

With an anti-bisphenol A scFv gene having the flexible linker (G4S)3 as a template, a VH gene fragment was amplified by PCR using NcoI-cc-VH sense (SEQ ID NO: 20) and .BIS A VH antisense (SEQ ID NO: 22), and a VL fragment was amplified by PCR using BIS A VL sense (SEQ ID NO: 23) and VL+NotI antisense (SEQ ID NO: 21). The PCR conditions were 25 cycles of denaturing at 94°C (15 sec); annealing (30 sec); and elongation at 68°C (30 sec). The annealing temperature was set at 56°C and 54°C for amplification of the VH gene fragment and the VL gene fragment, respectively. The amplified gene fragments were separated by agarose gel electrophoresis and purified, and then subjected to overlapping PCR as described below. KOD -Plus- (Toyobo) was used as a polymerase for PCR.

Table 1

| <Reaction mixtures><br>(i) | |
|---|---|
| 10× PCR buffer for KOD -Plus- | 5 μL |
| 2 mM dNTPs | 5 μL |
| 25 mM $MgSo_4$ | 2 μL |
| DNA (VH) | 1.5 μL |
| DNA (VL) | 1.5 μL |
| 10 pmol/μL linker DNA* | 1 μL |
| KOD -Plus- DNA polymerase | 1 μL |
| DIW | 33 μL |
| Total | 50 μL |
| | |
| (ii) | |
| 10× PCR buffer for KOD -Plus- | 5 μL |
| 2 mM dNTPs | 5 μL |
| 25 mM $MgSo_4$ | 2 μL |
| 10 pmol/μL NcoI-cc-VH sense primer | 1.5 μL |
| 10 pmol/μL VL+NotI antisense primer | 1.5 μL |
| KOD -Plus- DNA polymerase | 1 μL |
| DIW | 34 μL |
| Total | 50 μL |

Table 2

| <PCR conditions><br>(i) 10 cycles | | |
|---|---|---|
| Denaturing | 94°C | 30 sec |
| Annealing | 50°C | 30 sec |
| Elongation | 68°C | 30 sec |
| | | |
| (ii) 25 cycles | | |
| Denaturing | 94°C | 15 sec |
| Annealing | 55°C | 30 sec |
| Elongation | 68°C | 30 sec |

The reaction mixture (i) was prepared, and the reaction was performed for 10 cycles under PCR condition (i), then the reaction mixture (ii) was added and the reaction was performed for 25 cycles under PCR condition (ii).

After four rounds of panning, the plasmids were collected and infected into XL1-Blue cells. Twenty clones were selected from $7\times10^6$ clones and the nucleotide sequences of the genes encoding the polypeptide linkers in the plasmids were identified by a DNA sequencer. figure 2 shows the vector used for the scFv expression.

Among the 20 clones selected, 12 clones were found to be genetically deficient. Table 3 shows the amino acid sequences and the nucleotide sequences of the polypeptide linkers of the recovered scFv. Since an scFv with a polypeptide linker containing an amber stop codon (TAG) cannot be expressed in *E.coli* BL21(DE3)pLysS, the codon was changed to glutamine (CAA).

Table 3

| Linker No. | Amino acid sequence / Nucleotide sequence | SEQ ID NO: |
|---|---|---|
| No. 4 | GMGLSWAGQLPRFPQRASQG | 1 |
| | GGTATGGGTTTGTCTTGGGCTGGGCAGCTGCCTAGGTTTCCTCAGC<br>GTGCTAGTCAAGGG | 2 |
| No. 6 | KGRQQLQLCAPFDWCMFDAN | 3 |
| | AAGGGTCGGCAACAGCTGCAGTTGTGTGCGCCTTTTGATTGGTGTA<br>TGTTTGATGCTAAT | 4 |
| No. 10 | WVWSSRGQRSFRPSGRTVPL | 5 |
| | TGGGTTTGGAGTTCGCGGGGGCAGAGGTCTTTTCGGCCTTCGGGGC<br>GGACGGTGCCGCTT | 6 |
| No. 12 | KVVLWTTRVRDRGHTSTMWS | 7 |
| | AAGGTTGTTCTTTGGACTACGCGTGTTAGGGATAGGGGTCATACGT<br>CGACGATGTGGAGT | 8 |
| No. 14 | ADGHCHLKNFPLKPPPYFSV | 9 |
| | GCGGATGGGCATTGTCATCTGAAGAATTTTCCTTTGAAGCCTCCGC<br>CTTATTTTTCGGTT | 10 |

<Change in bacterial growth after IPTG induction due to differences in polypeptide linker nucleotide sequences>

Bacterial cell growth after IPTG induction was measured in cells with scFv selected by biopanning. Figure 3 shows the results. No. 2 is an scFv having a polypeptide linker of 13 amino acids due to a deficiency in the gene. From Figure 3 it can be seen that the growth of bacteria carrying linker No. 10, 12, and 14 increased in a range from 0.7 to 1.0 even after induction of expression with IPTG in the OD600 range of 5.5 to 5.7. On the other hand, in bacteria carrying linker No. 4, No. 6 or the flexible linker $(G_4S)_4$, the growth stopped after induction, and the final bacterial cell concentration at OD600 ranged from 1 to 2. As shown in Table 4, it was observed that one or more rare codons in *E. coli* were present in the nucleotide sequences encoding the linkers in the samples showing continued growth.

Table 4

| Linker No. | Nucleotide sequence (underline shows rare codons) | SEQ ID NO: |
|---|---|---|
| No. 4 | GGTATGGGTTTGTCTTGGGCTGGCAGCTGCCTAGGTTTCCTCAGC GTGCTAGTCAAGGG | 2 |
| No. 6 | AAGGGTCGGCAACAGCTGCAGTTGTGTGCGCCTTTTGATTGGTGTA TGTTTGATGCTAAT | 4 |
| No. 10 | TGGGTTTGGAGTTCGCGGGGGCAGAGGTCTTTTCGGCCTTCGGGGC GGACGGTGCCGCTT | 6 |
| No. 12 | AAGGTTGTTCTTTGGACTACGCGTGTTAGGGATAGGGGTCATACGT CGACGATGTGGAGT | 8 |
| No. 14 | GCGGATGGGCATTGTCATCTGAAGAATTTTCCTTTGAAGCCTCCGC CTTATTTTTCGGTT | 10 |

It is presumed that the translation rate of the scFv is temporarily slowed at the polypeptide linker due to the local presence of one to several rare codons in the polypeptide linker. Based on the above, high productivity of the target fusion protein can be expected if a DNA sequence is designed so that the translation rate in the linker of the fusion protein is temporarily delayed. Thus, as one example, an scFv expression vector was constructed having a linker $(LLKK)_4$ with a nucleotide sequence of 5'-CTACTAAAAAAACTACTAAAAAAACTACTAAAAAAACTACTAAAAAAA-3' (SEQ ID NO: 38) (underline indicates rare codons), and the bacterial growth rate after induction was measured. As a result, an extremely high growth rate was observed (also shown in Figure 3).

<Effect on productivity due to differences in linker polynucleotide sequence>

Productivity of scFv was compared in transformants that maintained growth after IPTG induction (linker No. 2, 10, 12, and 14) in a soluble and insoluble fractions. The expressed scFv was purified by the method shown below, and the amount of insoluble scFv and soluble scFv was determined in each fraction.

(1) The culture liquid was collected and centrifuged at 10,000g for 5 min to separate the culture supernatant and bacterial cell pellet.
(2) The bacterial cell pellet was suspended in 5 mL of lysis buffer (1% Triton X-100, 20 mM Tris-HCl (pH 8.0), 2 g/mL DNase, and 0.2 mg/mL lysozyme), and sonicated on ice using a probe-type sonicator for fifteen minutes.
(3) The sample was ultracentrifuged at 100,000g for 30 min at 4°C, and the supernatant was collected as an intracellular soluble fraction.
(4) The pellet was suspended in lysis buffer and washed twice by centrifugation at 23,000gr for 10 min.
(5) After washing, the pellet was suspended in distilled water, and the lysis buffer was removed by centrifugation twice under the same conditions as above.
(6) The resultant pellet was served as the intracellular insoluble fraction. The pellet was suspended in 1 mL of distilled water, and dried in a vacuum dryer.

(7) The pellet was weighed, and served as the weight of insoluble scFv.

(8) The intracellular soluble fraction was loaded onto a Ni-immobilized column, washed with 10 mM acetate buffer (containing 0.3 M NaCl, pH 6.0) followed by 0.2 mM phosphate buffer (containing 1 M NaCl, pH 7.2), and eluted with 0.5 M imidazole (pH 8.0).

(9) The level of scFv in the imidazole elution fraction was analyzed by SDS-PAGE.

(10) The assembly conformation of the scFv was analyzed by gel chromatography using 5-Diol-300-II (Nacalai Tesque).

(11) The imidazole elution fraction was loaded onto a bisphenol-immobilized column, washed with 10 mM acetate buffer (containing -0.3 M NaCl, pH 6.0), and eluted with 0.01 N NaOH (pH 12).

(12) The culture supernatant was loaded onto a bisphenol-immobilized column, washed with 10 mM acetate buffer (containing 0.3 M NaCl, pH 6.0), and eluted with 0.01 N NaOH (pH 12).

(13) The imidazole elution fraction and the eluate purified from the culture supernatant were dialyzed overnight against 10 mM acetate buffer (containing 0.3 M NaCl, pH 6.0), and the scFv concentration in both eluates was quantified by DC-protein assay. Bovine serum albumin (BSA) was used as a reference standard.

(14) The scFv amounts contained in the bacterial cells and in the culture supernatant were back-calculated from the scFv concentrations of both eluates, and the sum was considered to be the amount of soluble scFv expression.

Table 5

| Linker | Cell concentration (OD600) | Expression level (soluble) [mg/L] | Expression level (insoluble) [mg/L] | Soluble scFv/cell [mg/L-OD] | Insoluble scFv/cell [mg/L·OD] |
|---|---|---|---|---|---|
| $(G_4S)_4$ | 1.04 | 11.4 | 140 | 11.0 | 135 |
| No. 2 | 5.61 | 65.4 | 233 | 11.7 | 41.5 |
| No. 10 | 6.91 | 54.4 | 116 | 7.9 | 16.8 |
| No.12 | 7.42 | 60.3 | 212 | 8.1 | 28.6 |
| No. 14 | 5.56 | 39.3 | 220 | 7.1 | 39.6 |
| $(LLKK)_4$ | 8.23 | 38.6 | 204 | 4.7 | 24.7 |

As shown in Table 5, the productivity of a soluble fraction was approximately 3-fold to 6-fold greater than the productivity of scFv having a conventional flexible linker. The amount of expression of soluble scFv per unit bacterial cell showed roughly the same value regardless of the type of linker, while the amount of expression of the insoluble fraction (inclusion bodies) per unit bacterial cell of the scFv selected by biopanning was greatly suppressed. Therefore, it is believed that suppression of inclusion body formation affects cell growth properties after induction, and a final bacterial cell concentration and amount of expression were 5-fold to 8-fold higher than with the conventional flexible linker (G4S)4.

<Investigation of assembly conformation of selected scFv>

The soluble fraction of the selected scFv was purified by a Ni-chelate column, and the molecular weight was measured by gel chromatography. The molecular weights were 28 kDa for the scFv monomer and 56 kDa for the dimer, with the respective retention times of 23 min and 21 min. As shown in Figure 4, almost all the scFv formed dimers. To investigate whether this dimer formation was due to intermolecular interactions or to disulfide bounding, the dimer was analyzed by reducing and non-reducing SDS-PAGE procedures. As shown in Figure 5, when the scFv was denatured by SDS, the reduced form and the nonreduced form were both present as monomers, indicating that the scFv dimer was formed via intermolecular interactions.

<Antigen recognition properties of selected scFv>

The scFv purified by the Ni-chelate column was loaded onto an antigen binding column. After washing, the adsorbed scFv was eluted with 0.1 N hydrochloric acid. The antigen-recognizing activity of scFv was analyzed by SDS-PAGE for the Ni-chelate column purified fraction, the antigen binding column pass-through fraction and antigen binding column elution fraction. As shown in Figure 6, an scFv band was not seen only in the antigen binding column pass-through fraction, suggesting that almost all scFv molecules loaded onto the antigen binding column were adsorbed and eluted from the columns. Therefore, it was revealed that almost all the expressed scFv have antigen binding activity as an anti-bisphenol A antibody.

<Measurement of binding activity by biotinylated ELISA>

Next, the scFv were biotinylated, and its binding to bisphenol A coated on microtiter wells was detected using avidin HRP. As shown in Figure 7, each scFv showed a high signal in a dilution ratio-dependent manner. Therefore, scFv binding activity was also demonstrated by ELISA.

Example 2: Effect of modification by substitution of rare codon in nucleotide sequence encoding the anti-bisphenol A antibody fragment (scFv)

<Preparation of scFv containing polynucleotide sequence with rare codon>

An scFv gene having "minor (G4S)3" as a linker was prepared from the VH and VL gene fragments prepared by the method described in Example 1 and the minor (G4S)3 (SEQ ID NO: 24) linker DNA that encodes (G4S)3 containing rare codons. An scFv having "major No. 10" (SEQ ID NO: 25), a polypeptide linker encoding the same amino acid sequence as linker No. 10 but having no rare codon, was prepared in the same manner.

The resultant scFv genes were inserted in dephosphorylated pUC118/HincII, and selected by blue-white screening using XL1-Blue cells. The sequence of the scFv genes was confirmed by DNA sequencing. The pUC118 vector containing the sequence-verified scFv gene was treated with NcoI and NotI to excise the scFv genes. The target scFv genes were purified by agarose gel electrophoresis, and inserted into a pET22 vector pretreated with NcoI and NotI. *E. coli* XL1-Blue cells were transformed by the pET vector containing the scFv genes (pET-scFv), and those containing inserted gene were selected. Then *E. coli* BL21pLysS cells were transformed using the pET-scFv vectors recovered from XL1-Blue.

<Differences in productivity of scFv soluble molecules due to differences in polypeptide linker nucleotide sequence>

A vector was prepared containing pET22 as a backbone and an scFv gene having linker sequence (G4S)3, minor (G4S)3, No. 10, major No. 10, (LLKK)4, No. 4 or No. 14. *E. coli* strain BL21(DE3)pLysS cells transformed by the vector were cultured, and when growth reached the range of an OD600 value of 0.6 to 1.0, expression was induced with 0.1 mM IPTG. *E. coli* BL21(DE3)pLysS cells transformed by pET22 vector were used as a control. Time-course growth after induction was measured, and the produced amounts of soluble scFv and insoluble scFv were also measured. The amount of insoluble scFv was calculated based on the following formula.

$$\text{Insoluble scFv amount} = \text{total insoluble protein} - (64 \times \text{maximum cell concentration}/8.68)$$

wherein the value 64 represents the amount of insoluble protein when *E. coli* cells transformed by pET22 were cultured, and 8.68 represents the maximum bacterial cell concentration of E. coli transformed by pET22. The results are shown in Figure 8.

When minor (G4S)3 having rare codons was compared with (G4S)3 having no rare codon, the productivity of soluble scFv containing minor (G4S)3 was 88.0 mg/L, while the productivity of soluble scFv containing (G4S)3 was 57.4 mg/L. Similarly, when No. 10, a linker having rare codons, was compared with major No. 10 having no rare codon, the productivity of soluble scFv containing No. 10 was 116.0 mg/L, while the productivity of soluble scFv containing major No. 10 was 66.1 mg/L. It is clearly evidenced that the productivity of soluble scFv is significantly increased by introducing rare codons into the linker sequence.

The productivity of soluble scFv containing No. 4, which has one rare codon in the polynucleotide sequence encoding the polypeptide linker, was 63.7 mg/L, while the productivity of soluble scFv containing No. 14, which has two rare codons, was 87.7 mg/L, and the productivity of soluble scFv containing (LLKK)4, which has 8 rare codons, was 94.8 mg/L. These results indicate that the introduction of rare codons imparts a positive effect on the productivity of soluble scFv.

On the other hand, when minor (G4S)3 having rare codons was compared with (G4S)3 having no rare codon, the productivity of insoluble scFv containing minor (G4S)3 was 163 mg/L, while the productivity of insoluble scFv containing (G4S)3 was 185 mg/L. Similarly, when No. 10 containing rare codons was compared with major No. 10 having no rare codon, the productivity of insoluble scFv containing No. 10 was 43 mg/L, while the productivity of insoluble scFv containing major No. 10 was 71 mg/L. These results show an inverse correlation between the productivity of insoluble scFv and the soluble scFv as described above, indicating that the high productivity of soluble scFv having a linker containing rare codons is not due to an increase in total amount of scFv molecules produced, but is due to an increase in the ratio of soluble scFv.

<Differences in solubility of produced scFv due to differences in polypeptide linker nucleotide sequence>

To further examine the above results, the solubility of the produced scFv was calculated based on the following formula.

$$\text{Solubility} = 100 \times \text{amount of soluble scFv produced}/(\text{amount of soluble scFv produced} + \text{amount of insoluble scFv produced})$$

The results are shown in Figure 9.

When the minor (G4S)3 containing rare codons was compared with (G4S)3 having no rare codon, the solubility rate of the scFv containing minor (G4S)3 was 35.1%, while the solubility of scFv containing (G4S)3, was 23.7%. Similarly, when No. 10 containing rare codons was compared with major No. 10 having no rare codon, the solubility of scFv containing No. 10 was 62.2%, while the solubility of scFv containing major No. 10 was 56.8%. These results indicate that the high productivity of the expressed soluble scFv with a linker containing rare codons is not due to an increase in total amount of scFv molecules produced, but is due to an increase in the ratio of soluble scFv.

<Effect on host cell growth due to differences in polypeptide linker nucleotide sequence>

Figure 10 shows the analysis of the time-course of the cell growth after induction with 0.1 mM IPTG.

When minor (G4S)3 containing rare codons was compared with (G4S)3 having no rare codon, the bacterial cell concentration of transformants expressing scFv containing minor (G4S)3 was 5.07, while the bacterial cell concentration of transformants expressing scFv containing (G4S)3 was 2.58. Similarly, when No. 10 containing rare codons was compared with major No. 10 having no rare codon, the bacterial cell concentration of transformants expressing scFv containing No. 10 was 8.33, while the bacterial cell concentration of transformants expressing scFv having major No. 10 was 3.44. It is clearly evidenced that growth of transformants is significantly increased by introducing rare codons into the linker sequence.

The.bacterial cell concentration of transformants expressing scFv containing the linker No. 4, which has one rare codon in the polynucleotide sequence encoding the polypeptide linker, was 3.84, while the bacterial cell concentration of transformants expressing scFv containing the linker No. 14 having two rare codons was 8.66, and the bacterial cell concentration of transformants expressing scFv containing (LLKK)4 having 8 rare codons was 8.38. These results indicate that the introduction of one or more rare codons imparts a positive effect on the growth of transformant cells.

Example 3: Construction of MABL scFv, anti-CD47 antibody fragment

The VH gene fragment was amplified by PCR using VH-sense (SEQ ID NO: 26) and VH-antisense (SEQ ID NO: 27) primers and pCHOM2 (described in WO 2001/066737) as a template, and was purified by agarose gel electrophoresis.

Also the VL gene fragment was amplified using VL-sense (SEQ ID NO: 28) and VL-antisense (SEQ ID NO: 29) and the same template, and was purified by agarose gel electrophoresis.

Then overlapping PCR was performed using the VH gene fragment, VL gene fragment, and VH-(G4S)3-VL (SEQ ID NO: 30), with VH-sense (SEQ ID NO: 26) and VL-antisense (SEQ ID NO: 29) primers to prepare MABL scFv (G4S)3 gene containing no rare codon in the flexible linker gene.

Similarly, overlapping PCR was performed using the VH gene fragment, VL gene fragment, and VH-minor (G4S) 3-VL (SEQ ID NO: 31), with VH-sense (SEQ ID NO: 26) and VL-antisense : (SEQ ID NO: 29) primers to prepare MABL scFV-minor (G4S)3 gene containing rare codons in the flexible linker gene.

The MABL scFv(G4S)3scFv gene and MABL scFv-minor (G4S)3 gene were purified by agarose gel electrophoresis. Then a gene having restriction enzyme sites on both ends was amplified by PCR using 5'-end phosphorylated Nco VH-sense (SEQ ID NO: 32) primer and similarly phosphorylated Not VL-antisense (SEQ ID NO: 33) primer, using as a template either MABL scFv(G4S)3scFv or MABL scFv-minor (G4S) 3 gene.

Next, the amplified MABL scFv(G4S)3scFv gene and MABL scFv-minor (G4S)3 gene were purified by agarose gel electrophoresis, and ligated to pUC118/HincII (Takara Shuzo) that had been digested by HincII, and used for transformation of *E. coli.* The pUC118 vector containing either MABL scFv(G4S)3scFv gene or MABL scFv-minor (G4S)3 gene was selected by blue-white screening, and the insertion of the MABL scFv(G4S)3secFv gene or MABL scFv-minor (G4S)3 gene was confirmed.

Finally, the pUC118 vectors were digested by NcoI and NotI, and the MABL scFv(G4S)3 gene or MABL scFv-minor (G4S) 3 gene is purified by gel electrophoresis. They were inserted into pET22b(+) vectors that had been digested by NcoI and NotI to construct pET-MABL scFV and pET-minor MABL scFv vectors, respectively.

Example 4: Construction of MABL sc(Fv)2 and minor MABL sc(Fv)2 expression vectors

The Nco-MABL scFv gene having an NcoI on the 5' end was amplified by PCR using Nco-VH sense (SEQ ID NO: 32) and VL antisense (SEQ ID NO: 29) primers and using the pET-MABL scFv vector as a template, and was purified by agarose gel electrophoresis. Similarly, the MABL scFv-FLAG-Stop gene was amplified by PCR using VH sense (SEQ ID NO: 26) and VL-FLAG-Stop antisense (SEQ ID NO: 34) primers and using the pET-MABL scFv vector as a template, and was purified by agarose gel electrophoresis.

With the same method as above, the Nco-MABL minor scFv gene and the MABL minor scFv-FLAG-Stop gene were amplified by PCR using the pET-minor MABL scFv vector as a template, and purified by agarose gel electrophoresis.

The MABL sc(Fv)2 gene was amplified by overlapping PCR using the Nco-MABL scFv gene, MABL scFv-FLAG-Stop gene, VL-(G4S)3-VH gene (SEQ ID NO: 35), and Nco-VH sense (SEQ ID NO: 32) and Flag-Stop-Not antisense (SEQ ID NO: 36) primers. An NcoI site was inserted on the 5' end, and a FLAG peptide gene, Stop codon, and NotI site were inserted on the 3' end of the MABL sc(Fv)2 gene.

The MABL minor sc(Fv)2 gene was amplified by overlapping PCR by the same method as above using the Nco-MABL minor scFv gene, MABL minor scEv-FLAG-Stop gene, VL-(minor G4S)3-VH gene (SEQ ID NO: 37), and Nco-VH sense (SEQ ID NO: 32) and Flat-Stop-Not antisense (SEQ ID NO: 36) primers. An NcoI site was inserted on the 5' end, and a FLAG peptide gene, Stop codon, and NotI site were inserted on the 3' end of the MABL minor scr(Fv)2 gene.

The MABL sc(Fv)2 gene and MABL minor sc(Fv)2 gene were purified by agarose gel electrophoresis and cloned into pUC118 that had been digested by HincII. Clones containing the MABL sc(Fv)2 gene or MABL minor sc(Fv)2 gene were selected by blue-white screening, and insertion of the MABL sc(Fv)2 gene and MABL minor sc(Fv)2 gene was confirmed by DNA sequencing.

Next, the clones containing insertion were digested with NcoI and NotI, then the MABL sc(Fv)2 gene and MABL minor sc(Fv)2 gene were purified by gel electrophoresis, and inserted into pET22b(+) vectors that had been digested by NcoI and NotI. *E. coli* BL21(DE3)pLysS cells were then transformed by the pET-MABL sc(Fv)2 and pET-MABL minor sc(Fv)2 vectors.

Example 5: Production of MABL sc(Fv)2 and MABL minor sc(Fv)2 in *E. coli* transformed by MABL sc(Fv)2 and minor MABL sc(Fv)2 vectors

<Culture of transformed *E. coli*>

Single colonies of the aforementioned transformed *E. coli* were seeded into 4 mL of 2xYT medium containing ampicillin and chloramphenicol, and cultured overnight at 37°C and 160 rpm. These pre-cultured transformants were seeded into 50 mL of 2xYT medium in 500 mL baffle flasks at an OD600 of 0.1, and cultured at 37°C and 200 rpm until the OD600 value reached approximately 0.6 to 1.0.

After IPTG was added at a final concentration of 1 mM, culturing was continued for 7 hours at 30°C and 160 rpm. One mL of culture liquid was collected at each of the time points immediately before induction and at 1, 3, 5, and 7 hours after induction. The bacterial cell count was calculated from the OD600 value according to the following formula.

$$\text{Bacterial cell count [cells/L]} = \text{OD600} \times 1.6\times10^{11}$$

Figure 11 shows the bacterial cell growth curve after induction in the strains expressing MABL sc(Fv)2 and MABL minor sc(Fv)2. In the figure, the growth curves of the bacteria expressing each gene are represented as follows: squares are the control, circles are MABL sc(Fv)2, and triangles are MABL minor sc(Fv)2. As shown in Figure 11, even after the addition of IPTG, growth was not suppressed in the *E. coli* expressing MABL minor sc(Fv)2, which has rare codons in the DNA sequence of the flexible linker. On the other hand, bacterial growth stopped at approximately 3 hours after induction in the *E. coli* expressing MABL sc(Fv)2, which has a flexible linker having no rare codon. The strain expressing MABL minor sc(Fv)2 provided a bacterial cell amount of 2.5 times higher than the strain expressing MABL sc(Fv)2.

<Separation of culture supernatant, intracellular soluble fraction, and intracellular insoluble fraction>.

(1) The culture liquid was collected at 7 hours and centrifuged at 10,000g for 10 min at 4°C to separate the culture supernatant and the bacterial cell pellet.
(2) To the bacterial cell pellet was added 5 mL of lysis buffer (1% Triton X-100, 20 mM Tris-HCl (pH 8.0), 2 μg DNase, and 0.2 mg/mL lysozyme), and agitated for 20 min at 37°C.
(3) An Ultrasonic Disruptor DU-201 was set to OUTPUT 4, DUTY 60, and ultrasonication was performed for 15 min.
(4) The sample was ultracentrifuged at 600,000g for 30 min at 4°C.
(5) The obtained pellet was resuspended in 1 mL of lysis buffer and centrifuged at 23,500g for 5 min at 4°C.
(6) 500 μL of supernatant was removed, and then 500 μL of lysis buffer was added to the pellet.
(7) Steps (5) and (6) were repeated, then centrifuged at 23,500g for 5 min at 4°C.
(8) 500 μL of supernatant was removed and then 500 μL of distilled water was added to the pellet.
(9) The sample was centrifuged at 23,500g for 5 min at 4°C.
(10) Steps (8) and (9) were repeated, and 500 μL of supernatant was removed, then 500 μL of distilled water was added to the pellet.
(11) The contents from (10) were transferred to a pre-weighed Falcon tube, lyophilized overnight, and the tube was weighed again.
(12) The lyophilized sample was solubilized in 0.5 mL of 8 M urea and 10 mM mercaptoethanol, and used for SDS-PAGE and Western blot analysis.

The amount of expressed insoluble sc(Fv)2 antibody per unit bacterial cell obtained from *E. coli* BL21 (DE3) pLysS cells transformed by pET22 was $1.3 \times 10^{-10}$ mg. Based on this reference value, the amount of expressed sc(Fv)2 was calculated according to the following formula.

$$\text{Amount of expressed insoluble sc(Fv)2 [mg]} = (\text{measured weight of the pellet alone [mg]}) - (1.3 \times 10^{-10}\ \text{[mg/cell]} \times \text{final bacterial cell count})$$

Table 6

| Produced antibody fragment | Final bacterial cell concentration (cell/L) | Amount of insoluble fraction produced (mg/L) | Amount of insoluble sc(Fv)2 produced (mg/L) | Specific productivity of insoluble sc(Fv)2 fg-sc(Fv)2/cell |
|---|---|---|---|---|
| MABL sc(Fv)2 | $3.4 \times 10^{11}$ | 186 | 154.9 | 456 |
| MABL minor sc(Fv) 2 | $8.3 \times 10^{11}$ | 160 | 83.2 | 99 |
| Control (pET22b(+)) | $1.2 \times 10^{12}$ | 156 | 0 | 0 |

As shown in Table 6, the amount of insoluble MABL minor sc(Fv)2 produced was approximately half that of MABL sc(Fv)2. In addition, the amount of insoluble MABL minor sc(Fv)2 produced per bacterial cell was less than 1/4 that of MABL sc(Fv)2. These results indicate that the formation of inclusion bodies can be suppressed by inserting a flexible linker having rare codons into an scFv and sc(Fv)2.

<Fractionation of culture liquid before induction and at 1, 3, and 5 hours after induction>

After 1 mL of culture liquid collected before induction or at 1, 3, and 5 hours after induction was centrifuged at 15,000g for 10 min at 4°C, the bacterial cell pellet was treated with 100 μL per 1.0 OD of BugBuster (Novagen). The treated samples were centrifuged at 16,000g for 20 min at 4°C, and the supernatant was collected, which served as the intracellular soluble fraction. The pellet was solubilized in the same volume of 8 M urea, which served as the insoluble fraction.

<Western blot analysis>

(1) The culture supernatant, intracellular soluble fraction and insoluble fraction were diluted two-fold in sample buffer, and heated for 5 min at 94°C.
(2) 10 μL of each sample was applied to precast gel SuperSep 10-20% (Wako), and electrophoresed at 200 V for 1.5 hours.
(3) After electrophoresis, the proteins separated in the precast gel were transferred to a PVDF membrane (Millipore) using a tank-type trans-blotter (Bio-Rad) at 100 V for 1 hour.
(4) The PVDF membrane was blocked by incubating for 1 hour at room temperature in 5% Blocking One (Nacalai Tesque).
(5) After a wash in TBS, the membrane was immersed in a biotinylated anti-FLAG antibody solution diluted 1000-fold with TBS-T and incubated for 1 hour at room temperature.
(6) The membrane was washed 3 times with TBS-T for 5 min. each, then immersed in alkali phosphatase-labeled streptavidin solution diluted 2000-fold with TBS-T, and incubated for 1 hour at room temperature.
(7) After the membrane was washed 3 times with TSB-T for 5 min. each, BCIP/NTB solution was added, and the color development reaction was monitored.

<Evaluation of expressed MABL sc(Fv)2 and MABL minor sc(Fv)2 by SDS-PAGE>

The expression profile of MABL sc(Fv)2 and MABL minor sc(Fv)2 was analyzed by SDS-PAGE and Western blotting at the end of culturing. Figure 12 shows the results of SDS-PAGE analysis of MABL sc(Fv)2 and MABL minor sc(Fv)2 at 7 hours after expression induction. The molecular weight markers, in order, are 97, 66, 45, 31, 21, and 14 kDa. Lanes 1 to 3, 4 to 6, and 7 to 9 represent the intracellular soluble fraction, culture supernatant, and intracellular insoluble fraction, respectively. Lanes 1, 4, and 7 represent MABL sc(Fv)2.
Lanes 2, 5, and 8 represent MABL minor sc(Fv)2. Lanes 3, 6, and 9 represent the control. As shown in Figure 12, a protein band was detected in the insoluble fraction, which was not present in the control, indicating that a large part of both MABL minor sc(Fv)2 and MABL sc(Fv)2. were expressed as insoluble protein. A band with the predicted molecular weight was observed for MABL minor sc(Fv)2, and a band corresponding to approximately half (approximately 27 kDa) of the predicted molecular weight for MABL sc(Fv)2 was observed for MABL sc (Fv) 2.

<Evaluation of expressed MABL sc(Fv)2 and MABL minor sc(Fv)2 by Western blot>

Figure 13 shows the results of Western blot analysis of MABL sc(Fv)2 and MABL minor sc(Fv)2 at 7 hours after expression induction. The molecular weight markers, in order, are 97, 66, 45, 31, 21, and 14 kDa. Lanes 1 to 3, 4 to 6, and 8 to 10 represent the intracellular soluble fraction, culture supernatant, and intracellular insoluble fraction, respectively. Lane 7 is blank. Lanes 1, 4, and 8 represent MABL sc(Fv)2. Lanes 2, 5, and 9 represent MABL minor sc(Fv)2. Lanes 3, 6, and 10 represent the control. As shown in the Western blot analysis in Figure 13, bands were seen at both 55 kDa and 27 kDa in the insoluble fraction of MABL sc(Fv)2, indicating that MABL sc(Fv)2 undergoes protease degradation after expression. In contrast, MABL minor sc(Fv)2 showed a band at the predicted molecular weight.
The expression profile of sc(Fv)2 was analyzed by Western blot of each fraction before the addition of IPTG and at 1, 3, 5, and 7 hours after the addition. Figure 14 shows the time-course results of the intracellular insoluble fraction. The molecular weight markers, in order, are 97, 66, 45, 31, 21, and 14 kDa. Lanes 1 to 3, 4 to 6, 7 to 9, 10 to 12, and 13 to 15 represent pre-induction, 1 hour after induction, 3 hours after induction, 5 hours after induction, and 7 hours after induction; respectively. Lanes 1, 4, 7, 9, and 13 represent MABL sc(Fv)2. Lanes 2, 5, 8, 10, and 14 represent MABL minor sc(Fv)2. Lanes 3, 6, 9, 12, and 15 represent the control. As shown in Figure 14, MABL sc(Fv)2 and its degradation product were detected as inclusion bodies subsequent to 1 hour after induction. Even though bacterial cell growth had stopped, the amount of insoluble MABL sc(Fv)2 produced per unit cell increased as the culture time increased. MABL minor sc(Fv)2 was detected as inclusion bodies 3 hours after induction, and the amount of MABL minor sc(Fv)2 produced per unit cell was essentially constant starting 3 hours after induction.
Figure 15 shows the time-course results of the intracellular soluble fraction. The molecular weight markers, in order, are 97, 66, 45, 31, 21, and 14 kDa. Lanes 1 to 3, 4 to 6, 7 to 9, 10 to 12, and 13 to 15 represent pre-induction, 1 hour after induction, 3 hours after induction, 5 hours after induction, and 7 hours after induction, respectively. Lanes 1, 4, 7, 9, and 13 represent MABL sc(Fv)2. Lanes 2, 5, 8, 10, and 14 represent MABL minor sc(Fv)2. Lanes 3, 6, 9, 12, and 15 represent the control. As shown in the Western blot analysis of the soluble fraction presented in Figure 15, a band in the vicinity of 25 kDa was detected at 3, 5, and 7 hours after induction, which represent a degradation product of MABL sc(Fv)2. In contrast, MABL minor sc(Fv)2 showed a band at 1 and 3 hours after induction at the position corresponding to the predicted molecular weight.

Figure 16 shows the time-course results of the supernatant. The molecular weight markers, in order, are 97, 66, 45, 31, 21, and 14 kDa. Lanes 1 to 3, 4 to 6, 7 to 9, 10 to 12, and 13 to 15 represent pre-induction, 1 hour after induction, 3 hours after induction, 5 hours after induction, and 7 hours after induction, respectively. Lanes 1, 4, 7, 9, and 13 represent MABL sc(Fv)2. Lanes 2, 5, 8, 10, and 14 represent MABL minor sc(Fv)2. Lanes 3, 6, 9, 12, any 15 represent the control. As shown in Figure 16, the degradation product of MABL sc(Fv)2 was detected in the supernatant at 3, 5, and 7 hours after induction.

<Biacore analysis of MABL minor sc(Fv)2 and MABL sc(Fv)2 secreted into the supernatant>

Soluble human CD47 (hereinafter, referred to as shCD47) was expressed in CHO cells, with adding a FLAG tag to the C-terminus of the extracellular domain (amino acids 1 to 124) of CD47. The expressed shCD47 was purified using anti-FLAG M2 agarose (Sigma) as instructed in the manual.

A Biacore 3000 (Biacore) was used to measure antigen binding activity of MABL minor sc(Fv)2 and MABL sc(Fv)2, and HBS-EP buffer (Biacore) was used as the running buffer. An aldehyde group was inserted in the purified shCD47 sugar chain. shCD47 was immobilized on a CM5 sensor chip (Biacore) via the aldehyde group by an aldehyde coupling reaction according to the BIAapplications Handbook manual. To eliminate the effect of nonspecific binding, a cell that had not undergone any immobilization procedure was used as reference. The reference sensorgram was subtracted from the sensorgram of the cell having shCD47 immobilized. At a measurement flow rate of 5 μL/min, 5 μL of sample was injected. After binding, the chip was regenerated by injecting 5 μL of 10 mM HCl. The measurement was carried out with reference to the method of Kikuchi et al. (J. Biosci. Bioeng. (2005), 100, 311-317).

The dissociation constant (kd) was calculated from the slope of the dissociation phase using BIA evaluation ver. 3.1 (Biacore). The amount of MABL sc(Fv)2 in the sample was quantified from the amount of binding. In that process, a known concentration of MABL scFv dimer (diabody) expressed by CHO cells was used as a reference standard. Figure 17 shows a sensorgram produced by Biacore analysis of MABL sc(Fv)2 and MABL minor sc(Fv)2 in the culture supernatant at 7 hours after induction of expression, and Figure 18 shows a magnified view of that sensorgram. The solid line alone represents MABL sc(Fv)2 from CHO. The triangles and squares represent MABL sc(Fv)2 and MABL minor sc(Fv)2, respectively. X represents the control. As shown in Figure 17, the results of Biacore measurement of the culture supernatant demonstrated that both MABL minor sc(Fv)2 and MABL sc(Fv)2 are expressed as a molecule that binds with the antigen CD47.

The binding activities of MABL minor sc(Fv)2 and MABL sc(Fv)2 were kd = $3.9\times10^{-4}$ $s^{-1}$ and kd = $2.2\times10^{-3}$ $s^{-1}$, respectively. The dissociation rate of the dissociation phase of MABL minor sc(Fv)2 was slower, with approximately a 10-fold difference. The dissociation rate of MABL sc(Fv)2 from CHO was kd = $6.8\times10^{-5}$ $s^{-1}$. According to Kikuchi et al., there is a large difference in the dissociation rate constant toward CD47 between the monovalent antibody MABL scFv (kd = $3.9\times10^{-3}$ $s^{-1}$) and the divalent antibody MABL scFv dimer (diabody (kd = $7.1\times10^{-5}$ $s^{-1}$) (Biochem. Biophys. Res. Com. (2004) 315, 912-918). Thus it is believed that such an extremely slow dissociation rate of the diabody is caused by an avidity effect due to the divalent bonding.

Also in the Western blot analysis, a full-length band was found for MABL minor sc(Fv)2, suggesting that a molecule having divalent binding capability is mainly expressed. As a result, the dissociation rate becomes extremely slow, and no apparent difference was seen in the slope of the dissociation phase compared to the MABL scFv dimer (diabody) used as a control.

In contrast, it is believed that MABL sc(Fv)2 undergoes cleavage and the resultant fragments act as a monovalent antibody like an scFv, and shows approximately the same dissociation rate constant as the monovalent antibody scFv.

The expressed concentration of MABL minor sc(Fv)2 quantified from the amount of binding was approximately 0.15 mg/L in the culture supernatant, and approximately 0.17 mg/L in the intracellular soluble fraction.

## Claims

1. A process for producing an intracellular soluble fraction of an antibody fragment, where the antibody fragment comprises variable domains joined by a polypeptide linker or linkers which consist of 5 to 50 amino acids, said method comprising:

   (a) introducing, in the polynucleotide sequence encoding the linker(s), one or more rare codons, where a rare codon is one which has a tRNA ratio of 1.2% or less of the total tRNA in a prokaryotic host cell; and
   (b) culturing the prokaryotic host cell, which has been transformed by a vector comprising the polynucleotide encoding the antibody fragment inserted into the vector to allow the polynucleotide to be expressed in the host cell, and recovering the antibody fragment thus produced,

   where the sequence of the polynucleotide encoding the polypeptide linker(s) is such that when the mRNA transcribed from the polynucleotide is translated in the host cell transfected with the polynucleotide, the translation rate of the mRNA region encoding the polypeptide linker(s) is slower than the translation rate of the mRNA region encoding the variable domain immediately upstream thereof.

2. The process according to claim 1, wherein the prokaryotic cell is an *Escherichia coli* cell.

3. The process according to claim 1 or 2, wherein the rare codon is selected from GCC, CGG, AGG, CAA, CAC, CAT, CTA, CCC, CCA, and TCC.

4. The process according of claim 1 or 2, wherein the rare codon is selected from CGG, AGG, AGA, CTA, CCC, GGA, and ATA.

5. The process according to claim 1, wherein the polynucleotide encoding the linker(s) encodes an amino acid with a usage frequency which is in the top 10 lowest for an amino acid in the host cell transfected with the polynucleotide.

6. The process according to any one of claims 1 to 5, wherein the antibody fragment is scFv.

7. The process according to any one of claims 1 to 5, wherein the antibody fragment is sc(Fv)2.

8. The process according to any one of claims 1 to 3, wherein the polynucleotide encoding the polypeptide linker has a sequence set forth in SEQ ID NO: 6, 8, 10, 12, or 24.

## Patentansprüche

1. Verfahren zur Herstellung einer intrazellulären löslichen Fraktion eines Antikörperfragmentes, wobei das Antikörperfragment variable Domänen umfasst, die durch einen Polypeptid-Linker oder -Linker, bestehend aus 5 bis 50 Aminosäuren, verbunden sind, wobei das Verfahren umfasst:

   (a) Einführen in die Polynucleotidsequenz, die den (die) Linker codiert, eines oder mehrerer seltenen Codon(s), wobei ein seltenes Codon eines ist, das einen tRNA-Anteil von 1,2% oder weniger an der gesamten tRNA in einer prokaryotischen Wirtszelle hat, und

(b) Kultivieren der prokaryotischen Wirtszelle, welche durch einen Vektor transformiert worden ist, der das Polynucleotid, das das Antikörperfragment codiert, in den Vektor eingesetzt umfasst, um zu erlauben, dass das Polynucleotid in der Wirtszelle exprimiert wird, und Gewinnen des so produzierten Antikörperfragments,

wobei die Sequenz des Polynucleotids, das den Polypeptid-linker (die Polypeptidlinker) codiert, so ist, dass, wenn die mRNA, die von dem Polynucleotid transkribiert wurde, in der Wirtszelle translatiert wird, die mit dem Polynucleotid transfiziert ist, die Translationsrate der mRNA-Region, die den Polypeptidlinker (die Polypeptidlinker) codiert, langsamer ist als die Translationsrate der mRNA-Region, die die variable Domäne unmittelbar stromaufwärts davon codiert.

2. Verfahren nach Anspruch 1, wobei die prokaryotische Zelle eine *Escherichia-coli*-Zelle ist.

3. Verfahren gemäß Anspruch 1 oder 2, wobei das seltene Codon aus GCC, CGG, AGG, CAA, CAC, CAT, CTA, CCC, CCA und TCC ausgewählt wird.

4. Verfahren nach Anspruch 1 oder 2, wobei das seltene Codon aus CGG, AGG, AGA, CTA, CCC, GGA und ATA ausgewählt wird.

5. Verfahren nach Anspruch 1, wobei das Polynucleotid, das den (die) Linker codiert, eine Aminosäure mit einer Verwendungshäufigkeit, die in den Top-10-untersten für eine Aminosäure in der Wirtszelle ist, die mit dem Polynucleotid transfiziert ist, codiert.

6. Verfahren gemäß einem der Ansprüche 1 bis 5, wobei das Antikörperfragment scFv ist.

7. Verfahren gemäß einem der Ansprüche 1 bis 5, wobei das Antikörperfragment sc(Fv)2 ist.

8. Verfahren gemäß einem der Ansprüche 1 bis 3, wobei das Polynucleotid, das den Polypeptidlinker codiert, eine Sequenz hat, die in SEQ ID NO: 6, 8, 10, 12 oder 24 angegeben ist.

## Revendications

1. Procédé de préparation d’une fraction soluble intracellulaire d’un fragment d’anticorps, lequel fragment d’anticorps comporte des domaines variables reliés par un ou des raccord(s) polypeptidique(s) constitué(s) de 5 à 50 résidus d’acides aminés, lequel procédé comporte les étapes suivantes :

a) introduire, dans la séquence polynucléotidique codant le ou les raccord(s), un ou plusieurs codon(s) rare(s), étant entendu qu’un codon rare est un codon auquel correspond un ARNt qui représente 1,2 % ou moins de la quantité totale d’ARNt présente dans une cellule hôte procaryote ;

b) et cultiver une cellule hôte procaryote qui a été transformée avec un vecteur comportant, inséré dans le vecteur, le polynucléotide codant le fragment d’anticorps, pour permettre au polynucléotide d’être exprimé dans la cellule hôte, et récupérer le fragment d’anticorps ainsi préparé ;

et dans lequel la séquence du polynucléotide codant le ou les raccord(s) polypeptidique(s) est telle que, quand l’ARNm transcrit à partir de ce polynucléotide est traduit dans la cellule hôte transfectée avec ce polynucléotide, la vitesse de traduction de la région d’ARNm codant le ou les raccord(s) polypeptidique(s) est plus faible que la vitesse de traduction de la région d’ARNm codant le domaine variable immédiatement en amont.

2. Procédé conforme à la revendication 1, dans lequel la cellule procaryote est une cellule *d’Escherichia coli.*

3. Procédé conforme à la revendication 1 ou 2, dans lequel le codon rare est choisi parmi les codons GCC, CGG, AGG, CAA, CAC, CAT, CTA, CCC, CCA et TCC.

4. Procédé conforme à la revendication 1 ou 2, dans lequel le codon rare est choisi parmi les codons CGG, AGG, AGA, CTA, CCC, GGA et ATA.

5. Procédé conforme à la revendication 1, dans lequel le polynucléotide codant le ou les raccord(s) code un acide aminé dont la fréquence d'utilisation se trouve parmi les 10 plus basses pour un acide aminé, dans la cellule hôte transfectée avec le polynucléotide.

6. Procédé conforme à l'une des revendications 1 à 5, dans lequel le fragment d'anticorps est un fragment scFv.

7. Procédé conforme à l'une des revendications 1 à 5, dans lequel le fragment d'anticorps est un fragment $sc(Fv)_2$.

8. Procédé conforme à l'une des revendications 1 à 3, dans lequel le polynucléotide codant le raccord polypeptidique présente la séquence donnée en tant que Séquence N° 6, 8, 10, 12 ou 24.

【Fig.1】

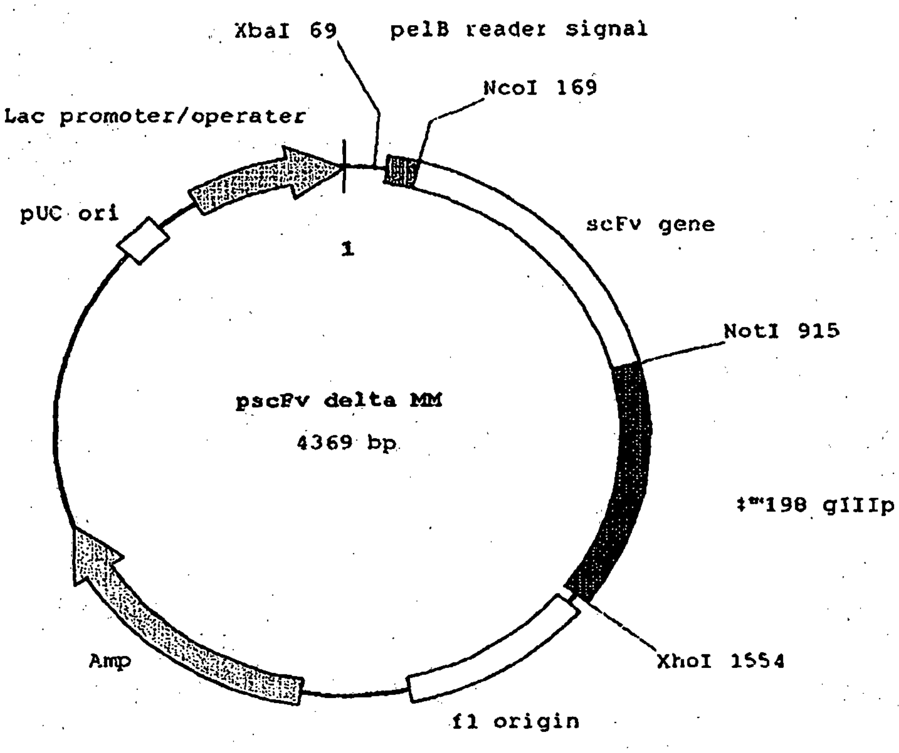


Plasmid name:pscFv delta MM
Plasmid size:4369 bp

【Fig.2】

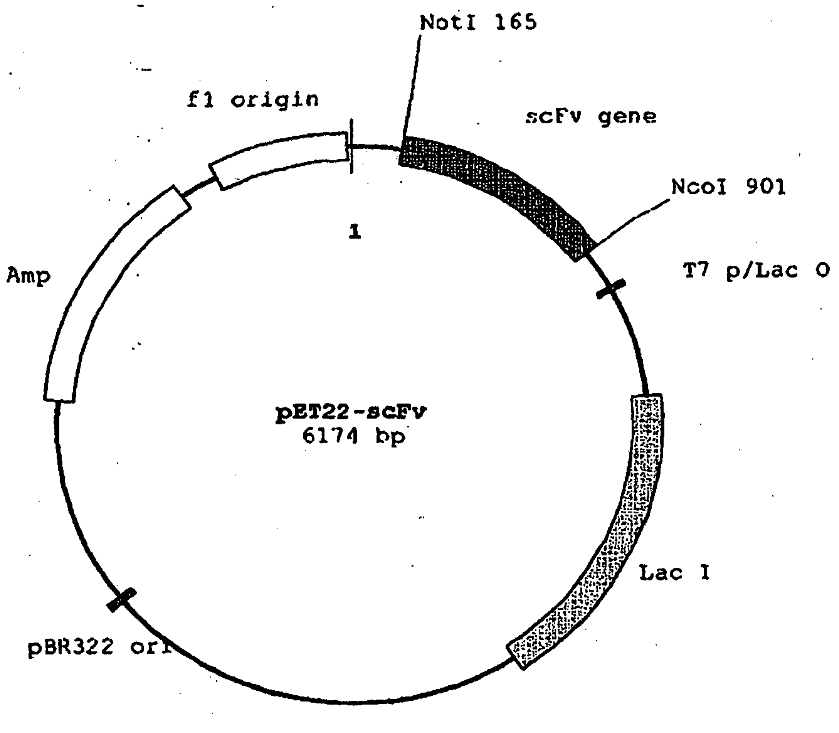


Plasmid name:pET22-scFv
Plasmid size:6174 bp

【Fig.3】

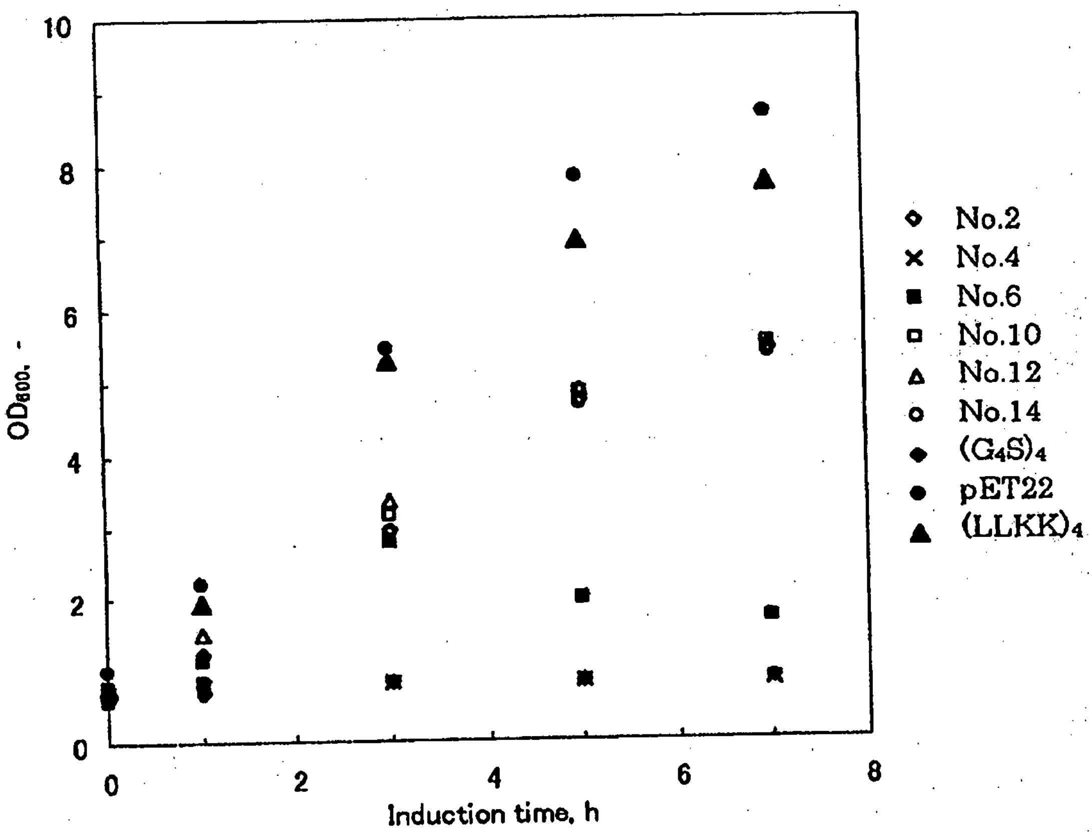
10
8
6
4
2
0
OD600, -
0
2
4
6
8
Induction time, h
No.2
No.4
No.6
No.10
No.12
No.14
(G4S)4
pET22
(LLKK)4

【Fig.4】

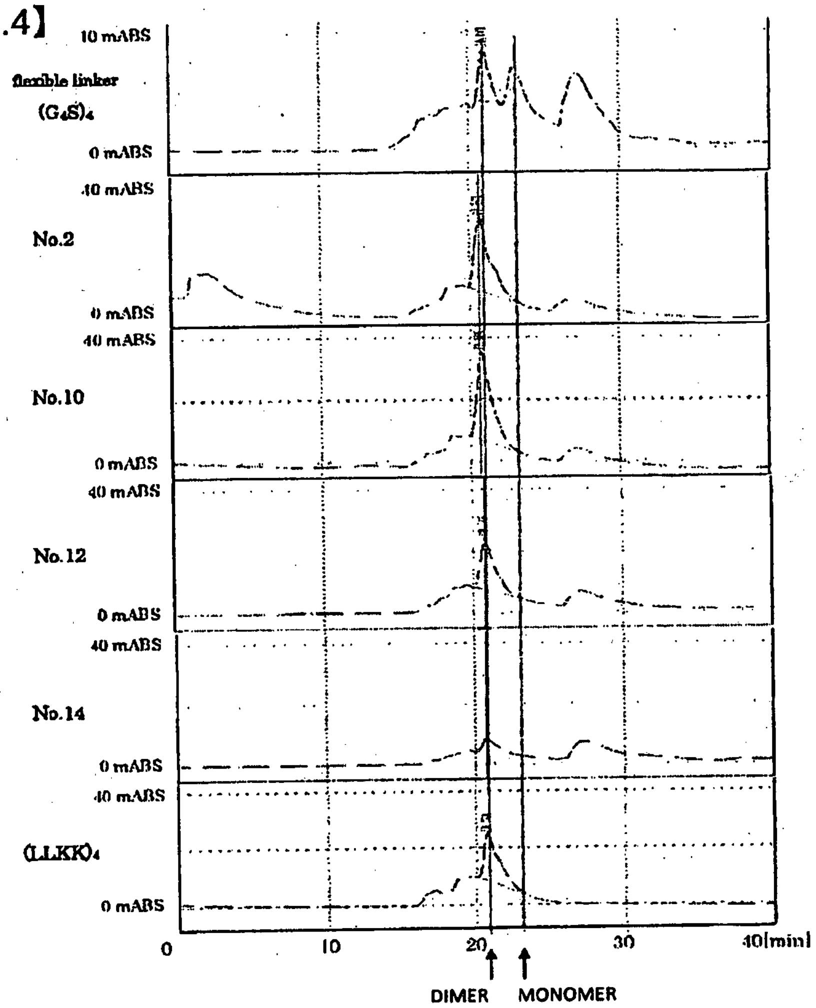
10 mABS
flexible linker
$(G_4S)_4$
0 mABS
40 mABS
No.2
0 mABS
40 mABS
No.10
0 mABS
40 mABS
No.12
0 mABS
40 mABS
No.14
0 mABS
40 mABS
$(LLKK)_4$
0 mABS
0
10
20
30
40[min]
DIMER
MONOMER

【Fig.5】

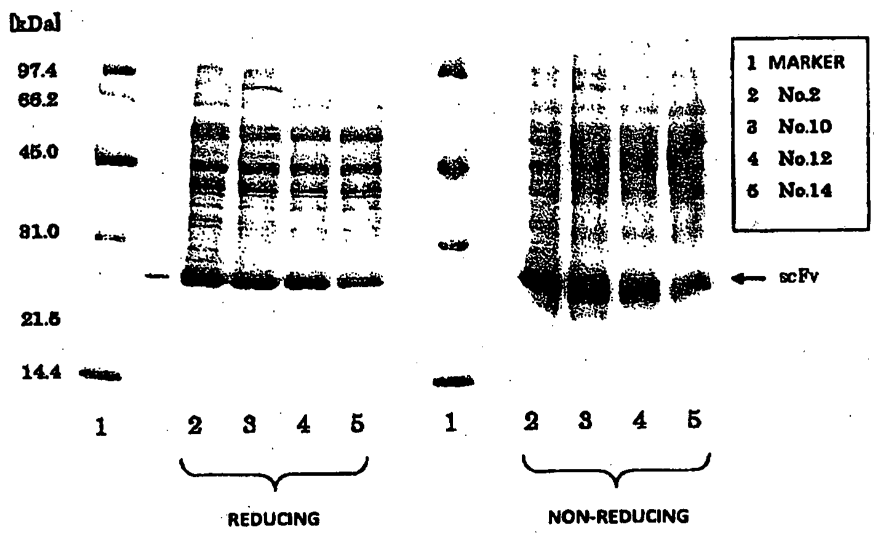
[kDa]
97.4
66.2
45.0
31.0
21.5
14.4
1 MARKER
2 No.2
3 No.10
4 No.12
5 No.14
scFv
1
2
3
4
5
1
2
3
4
5
REDUCING
NON-REDUCING

【Fig.6】

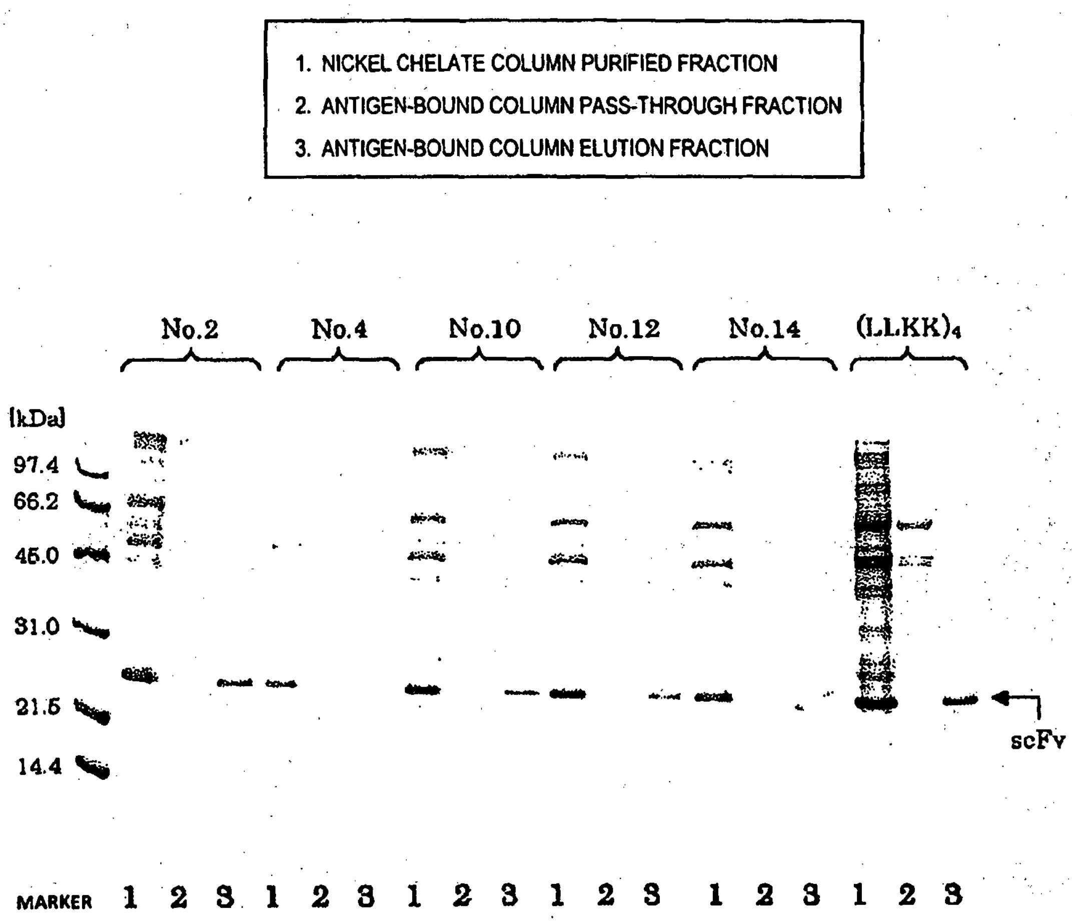
1. NICKEL CHELATE COLUMN PURIFIED FRACTION
2. ANTIGEN-BOUND COLUMN PASS-THROUGH FRACTION
3. ANTIGEN-BOUND COLUMN ELUTION FRACTION
No.2
No.4
No.10
No.12
No.14
(LLKK)4
[kDa]
97.4
66.2
45.0
31.0
21.5
14.4
scFv
MARKER 1 2 3 1 2 3 1 2 3 1 2 3 1 2 3 1 2 3

【Fig.7】

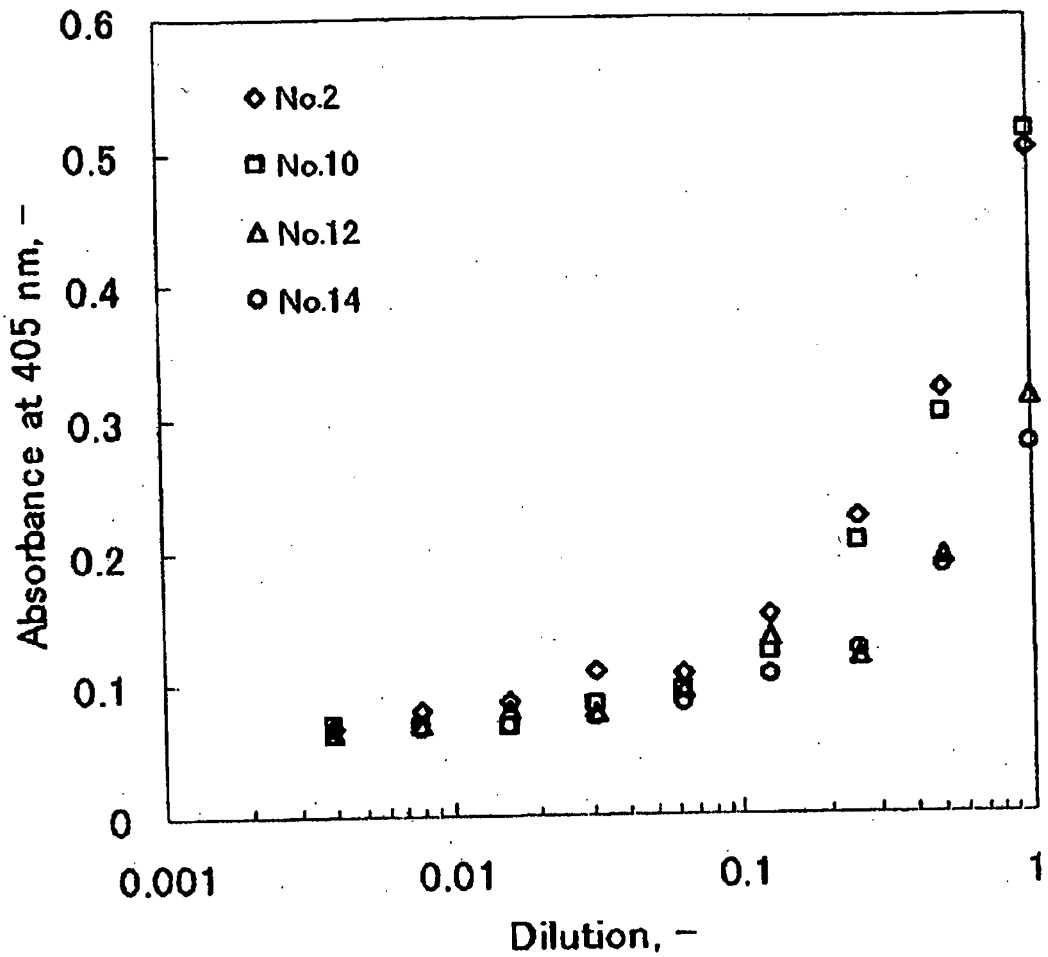


COATING: BISPHENOL A-BSA CONJUGATE, 10 μg/mL

SAMPLE: BIOTINYLATED SCFV SAMPLE

LABEL: STREPTAVIDIN-HRP CONJUGATE

【Fig.8】

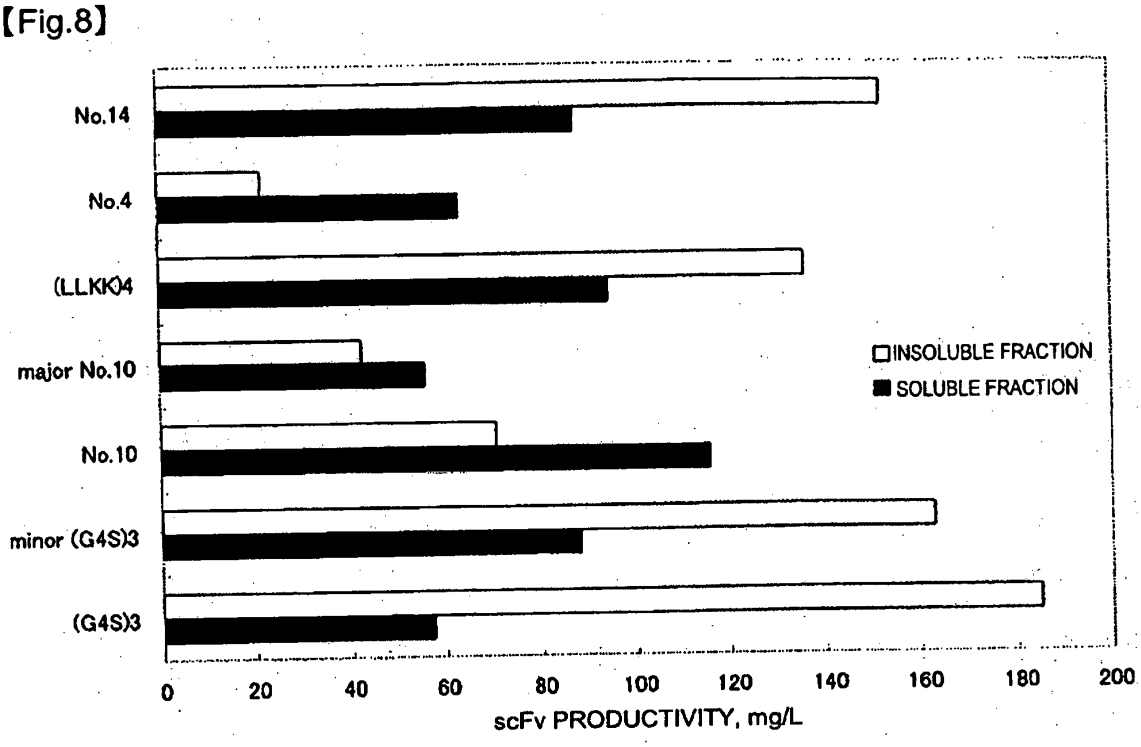
No.14
No.4
(LLKK)4
major No.10
No.10
minor (G4S)3
(G4S)3
INSOLUBLE FRACTION
SOLUBLE FRACTION
0
20
40
60
80
100
120
140
160
180
200
scFv PRODUCTIVITY, mg/L

【Fig.9】

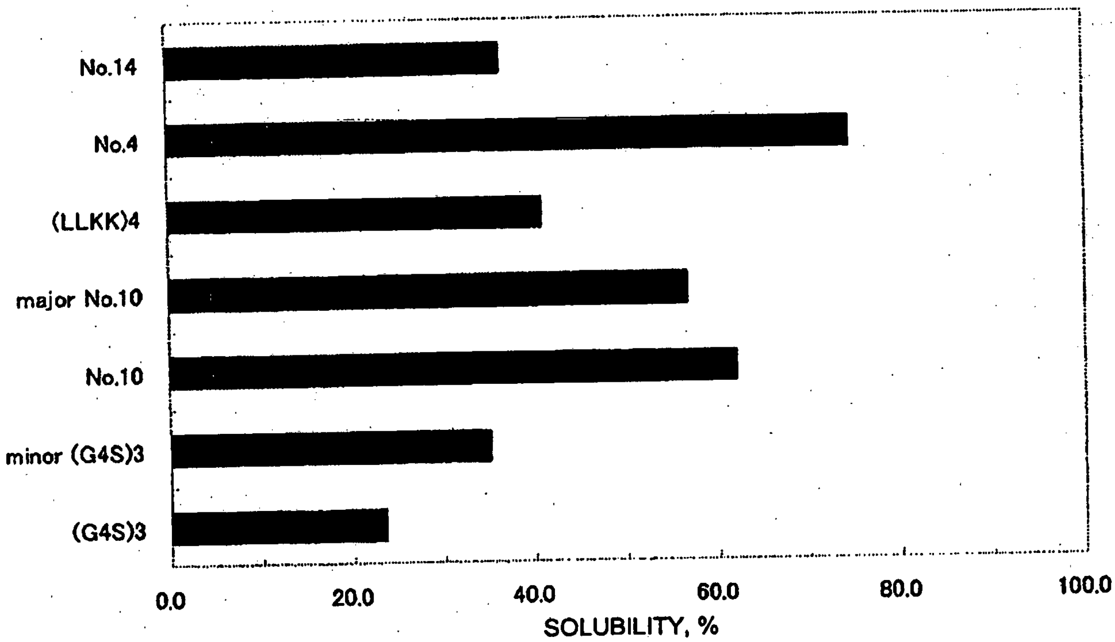
No.14
No.4
(LLKK)4
major No.10
No.10
minor (G4S)3
(G4S)3
0.0
20.0
40.0
60.0
80.0
100.0
SOLUBILITY, %

【Fig.10】

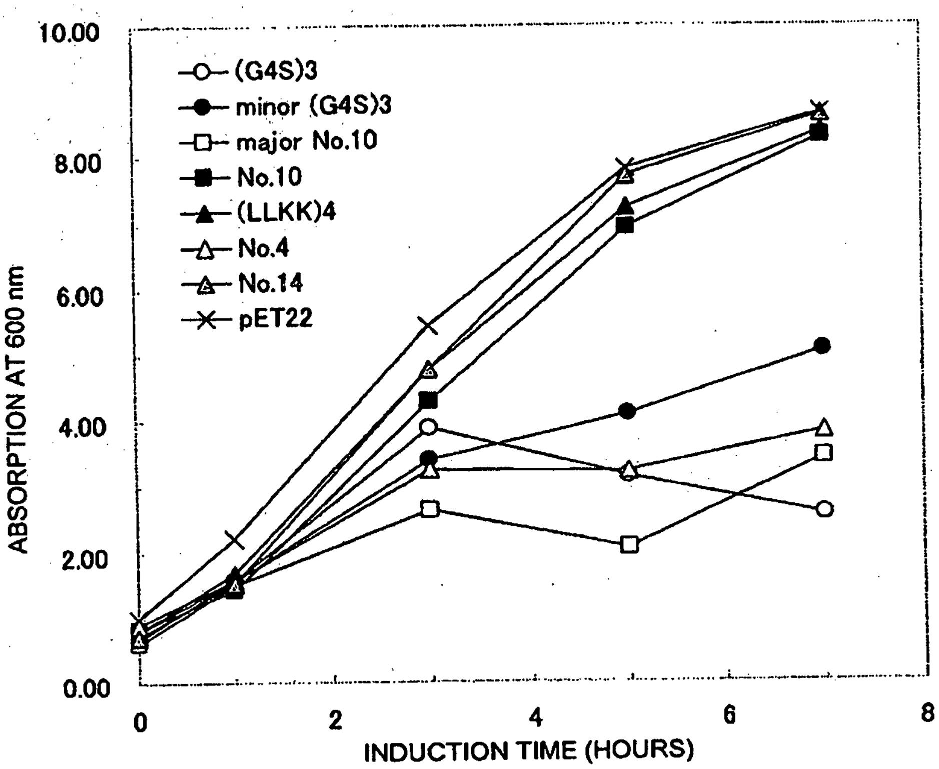
(G4S)3
minor (G4S)3
major No.10
No.10
(LLKK)4
No.4
No.14
pET22
ABSORPTION AT 600 nm
10.00
8.00
6.00
4.00
2.00
0.00
0
2
4
6
8
INDUCTION TIME (HOURS)

【Fig.11】

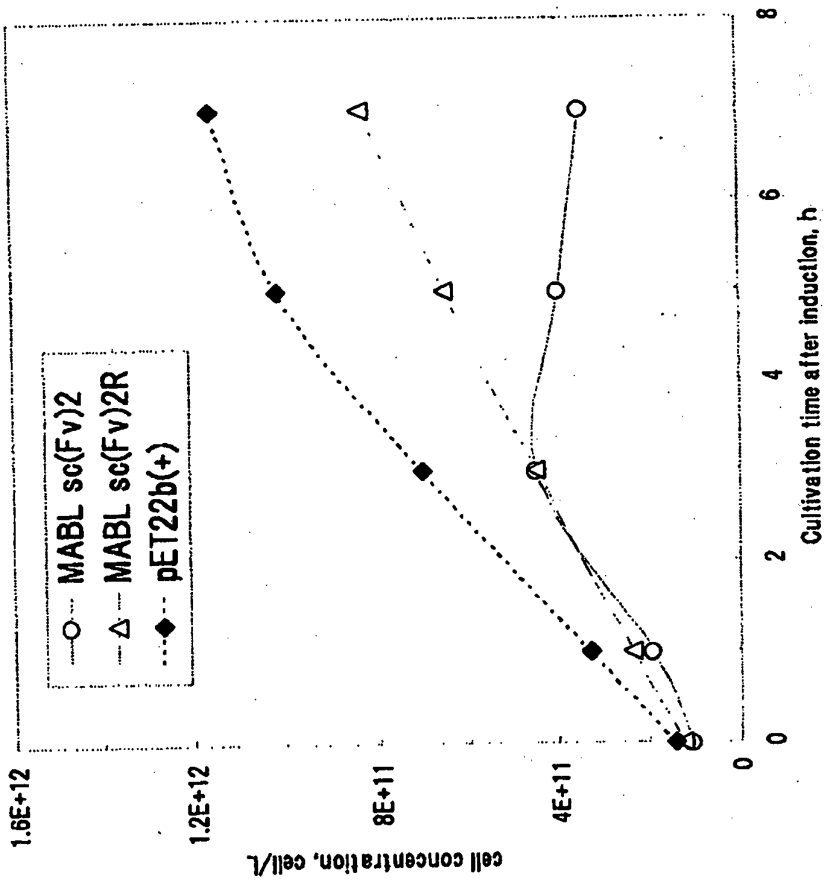
MABL sc(Fv)2
MABL sc(Fv)2R
pET22b(+)
cell concentration, cell/L
1.6E+12
1.2E+12
8E+11
4E+11
0
0
2
4
6
8
Cultivation time after induction, h

【Fig.12】

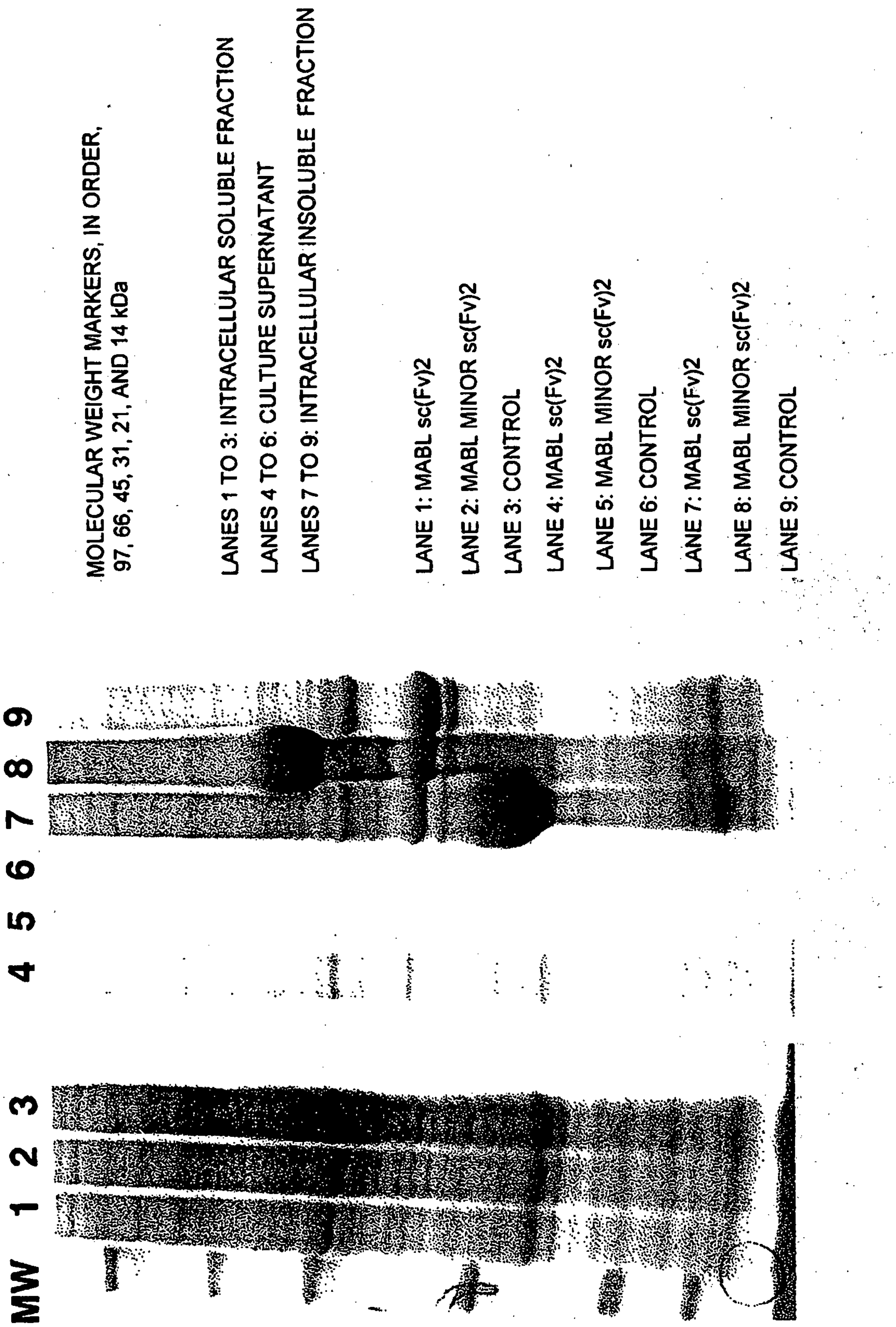
MW 1 2 3 4 5 6 7 8 9
MOLECULAR WEIGHT MARKERS, IN ORDER, 97, 66, 45, 31, 21, AND 14 kDa
LANES 1 TO 3: INTRACELLULAR SOLUBLE FRACTION
LANES 4 TO 6: CULTURE SUPERNATANT
LANES 7 TO 9: INTRACELLULAR INSOLUBLE FRACTION
LANE 1: MABL sc(Fv)2
LANE 2: MABL MINOR sc(Fv)2
LANE 3: CONTROL
LANE 4: MABL sc(Fv)2
LANE 5: MABL MINOR sc(Fv)2
LANE 6: CONTROL
LANE 7: MABL sc(Fv)2
LANE 8: MABL MINOR sc(Fv)2
LANE 9: CONTROL

【Fig.13】

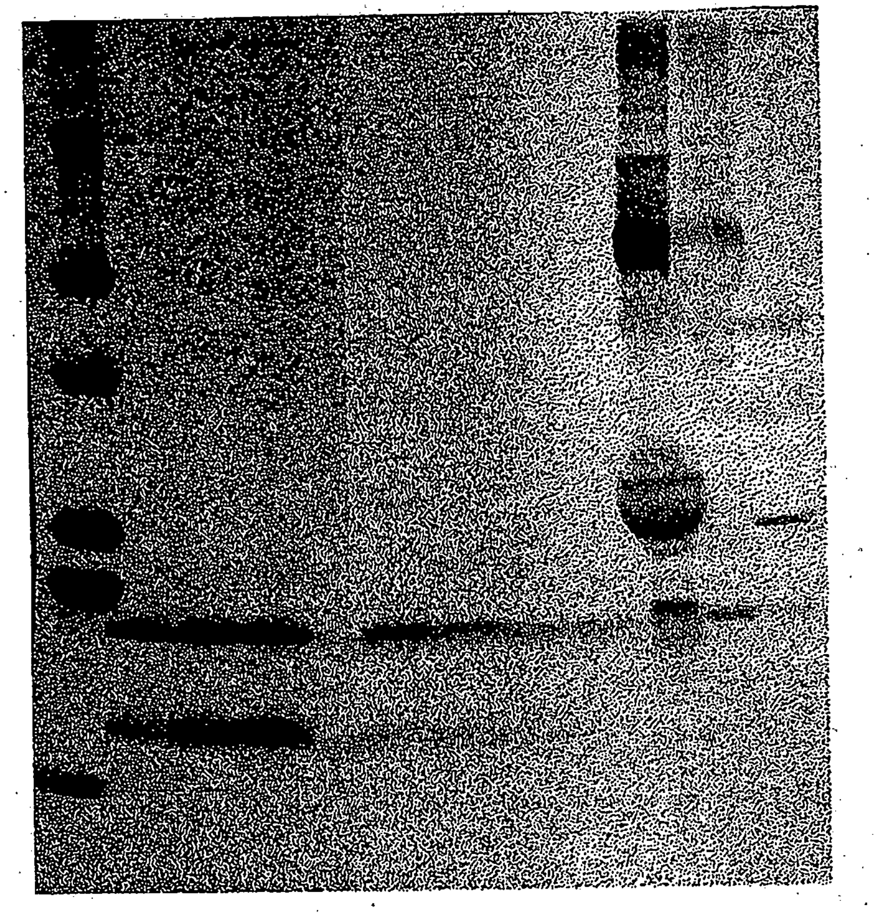


MOLECULAR WEIGHT MARKERS, IN ORDER, 97, 66, 45, 31, 21, AND 14 kDa

LANES 1 TO 3: INTRACELLULAR SOLUBLE FRACTION
LANES 4 TO 6: CULTURE SUPERNATANT
LANE 7: BLANK
LANES 8 TO 10: INTRACELLULAR INSOLUBLE FRACTION

LANE 1: MABL sc(Fv)2
LANE 2: MABL MINOR sc(Fv)2
LANE 3: CONTROL
LANE 4: MABL sc(Fv)2
LANE 5: MABL MINOR sc(Fv)2
LANE 6: CONTROL
LANE 8: MABL sc(Fv)2
LANE 9: MABL MINOR sc(Fv)2
LANE 10: CONTROL

【Fig.14】

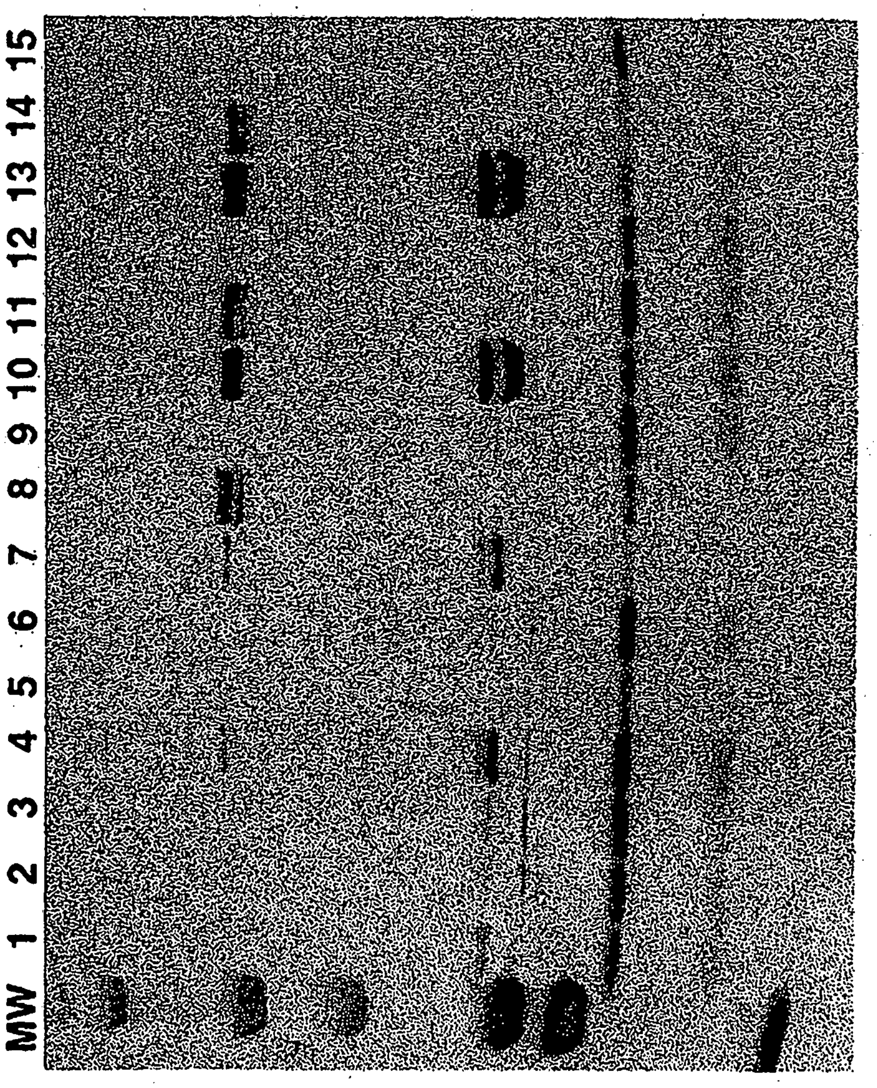


MOLECULAR WEIGHT MARKERS, IN ORDER, 97, 66, 45, 31, 21, AND 14 kDa

LANES 1 TO 3: PRE-INDUCTION

LANES 4 TO 6: 1 HOUR AFTER INDUCTION

LANES 7 TO 9: 3 HOURS AFTER INDUCTION

LANES 10 TO 12: 5 HOURS AFTER INDUCTION

LANES 13 TO 15: 7 HOURS AFTER INDUCTION

LANES 1, 4, 7, 9, AND 13: MABL sc(Fv)2

LANES 2, 5, 8, 10, AND 14: MABL MINOR sc(Fv)2

LANES 3, 6, 9, 12, AND 15: CONTROL

【Fig.15】

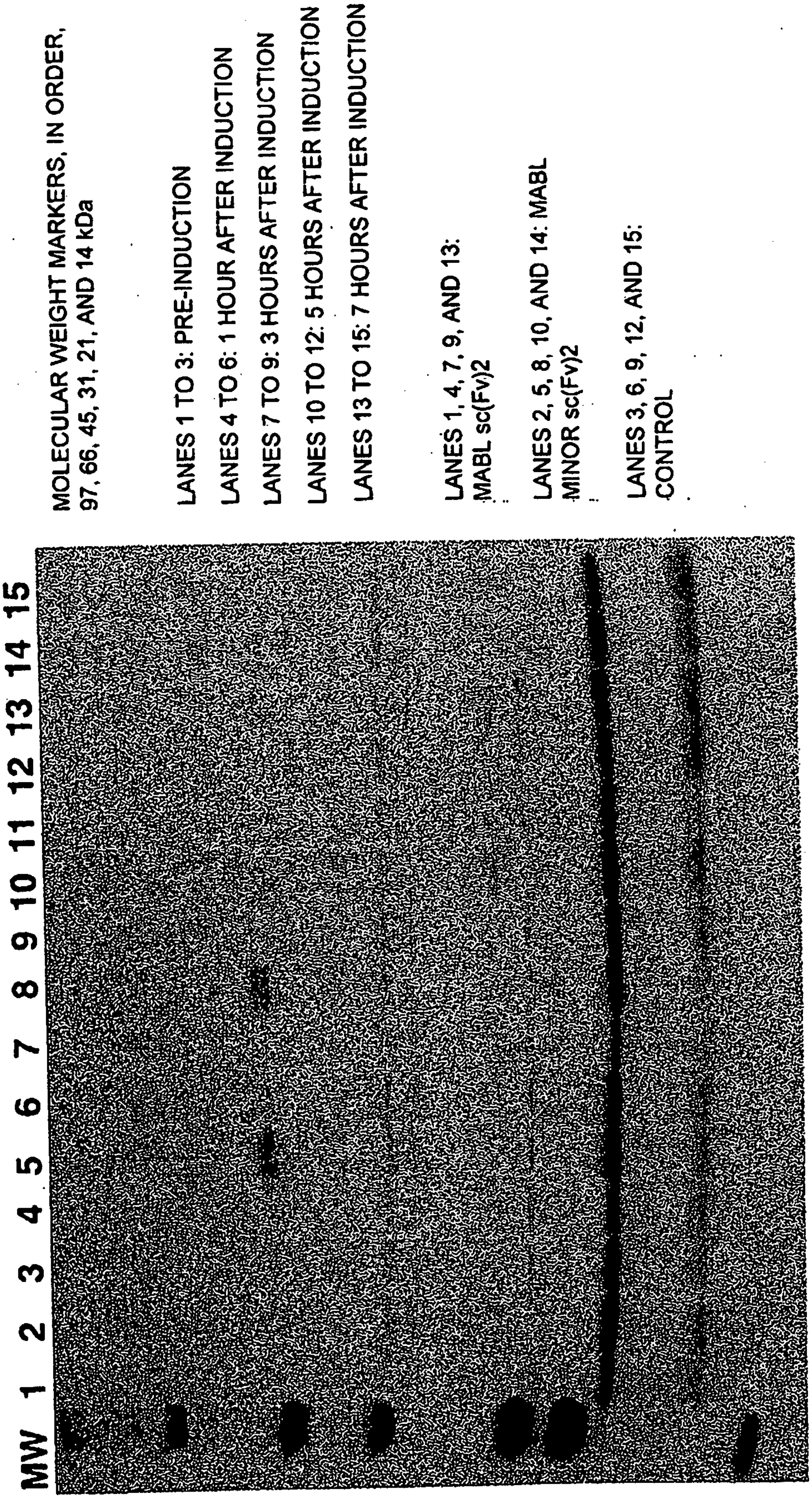
MOLECULAR WEIGHT MARKERS, IN ORDER, 97, 66, 45, 31, 21, AND 14 kDa
LANES 1 TO 3: PRE-INDUCTION
LANES 4 TO 6: 1 HOUR AFTER INDUCTION
LANES 7 TO 9: 3 HOURS AFTER INDUCTION
LANES 10 TO 12: 5 HOURS AFTER INDUCTION
LANES 13 TO 15: 7 HOURS AFTER INDUCTION
LANES 1, 4, 7, 9, AND 13: MABL sc(Fv)2
LANES 2, 5, 8, 10, AND 14: MABL MINOR sc(Fv)2
LANES 3, 6, 9, 12, AND 15: CONTROL
MW 1 2 3 4 5 6 7 8 9 10 11 12 13 14 15

【Fig.16】

MOLECULAR WEIGHT MARKERS, IN ORDER,
97, 66, 45, 31, 21, AND 14 kDa

LANES 1 TO 3: PRE-INDUCTION
LANES 4 TO 6: 1 HOUR AFTER INDUCTION
LANES 7 TO 9: 3 HOURS AFTER INDUCTION
LANES 10 TO 12: 5 HOURS AFTER INDUCTION
LANES 13 TO 15: 7 HOURS AFTER INDUCTION

LANES 1, 4, 7, 9, AND 13:
MABL sc(Fv)2

LANES 2, 5, 8, 10, AND 14: MABL
MINOR sc(Fv)2

LANES 3, 6, 9, 12, AND 15:
CONTROL

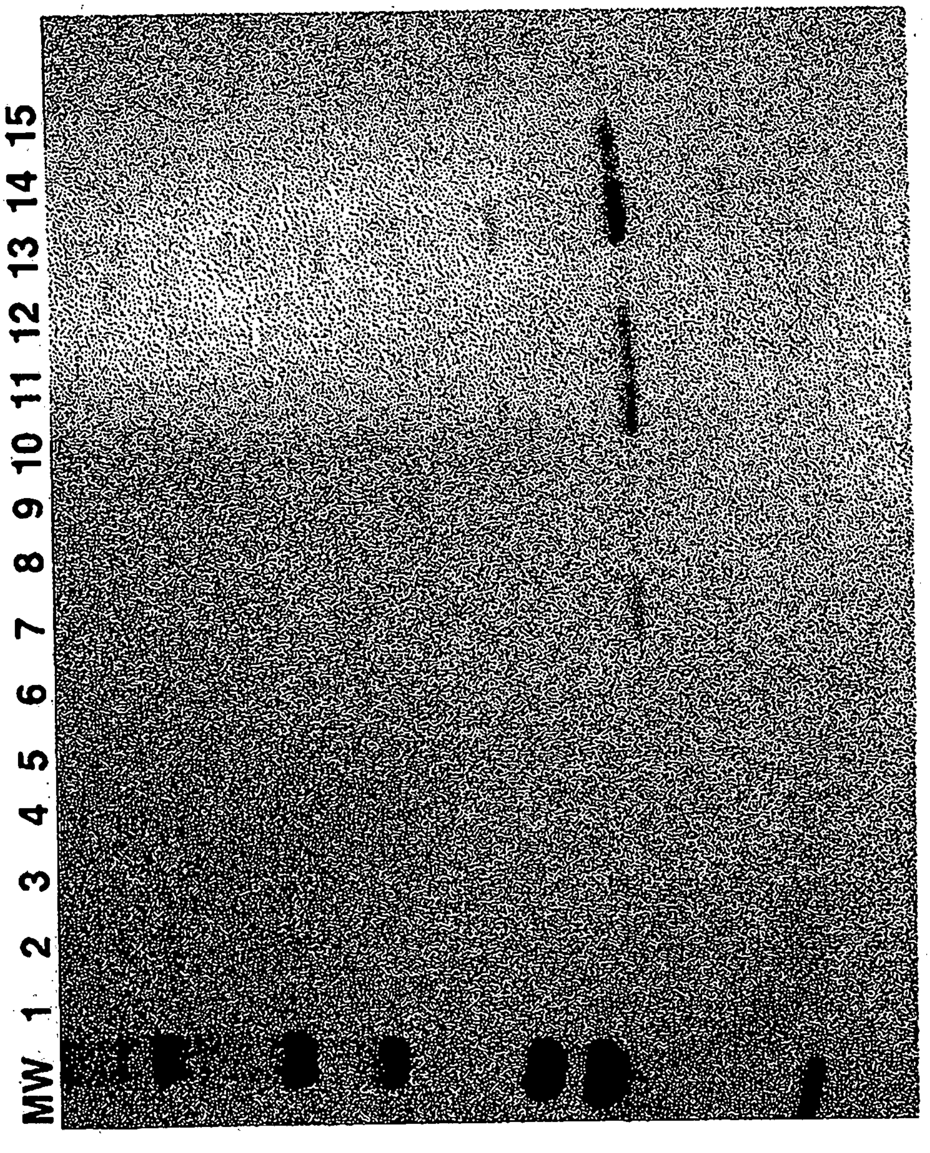

【Fig.17】

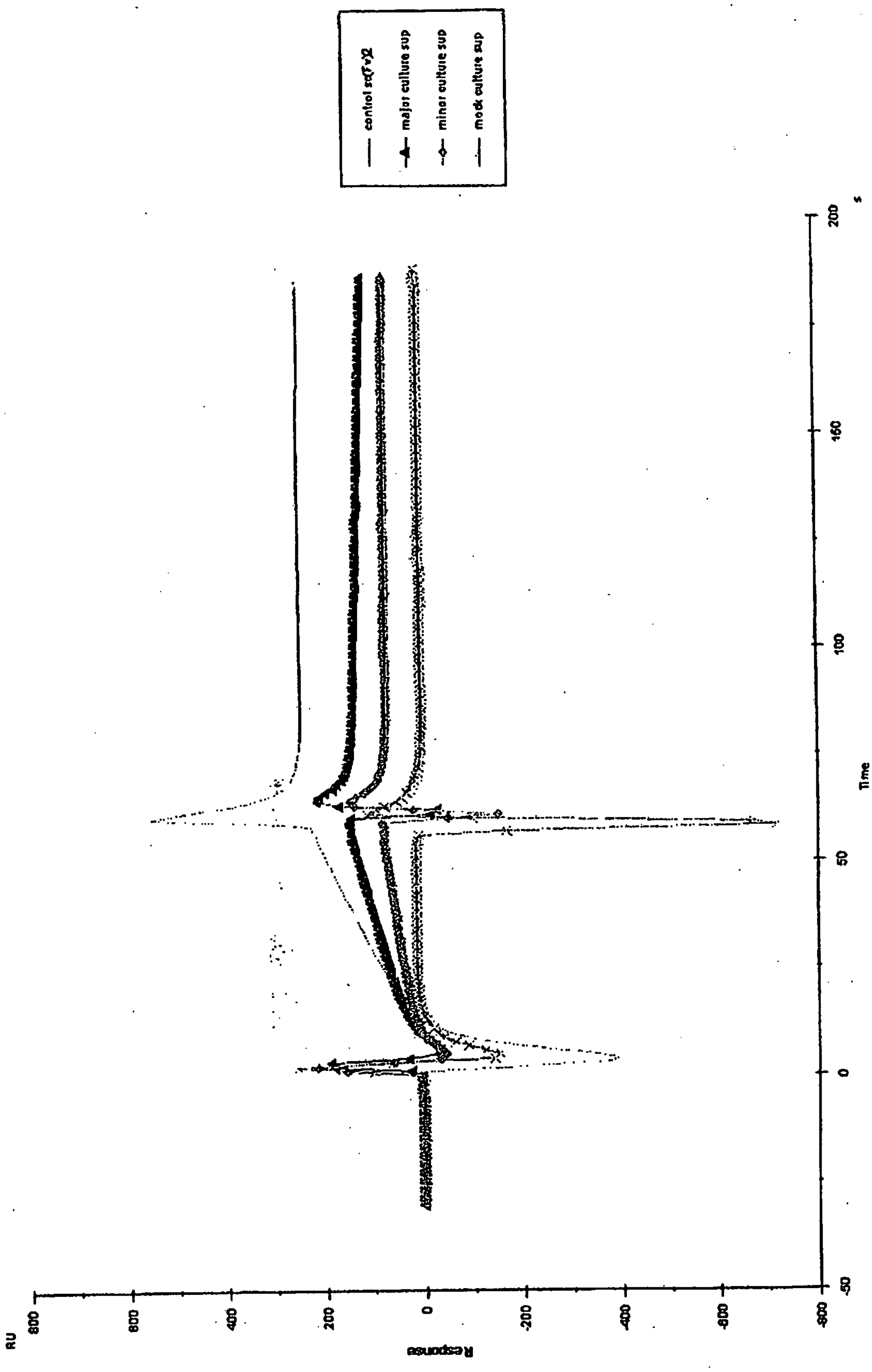
control sc(Fv)2
major culture sup
minor culture sup
mock culture sup
RU
Response
Time
s
800
600
400
200
0
-200
-400
-600
-800
-50
0
50
100
150
200

【Fig.18】

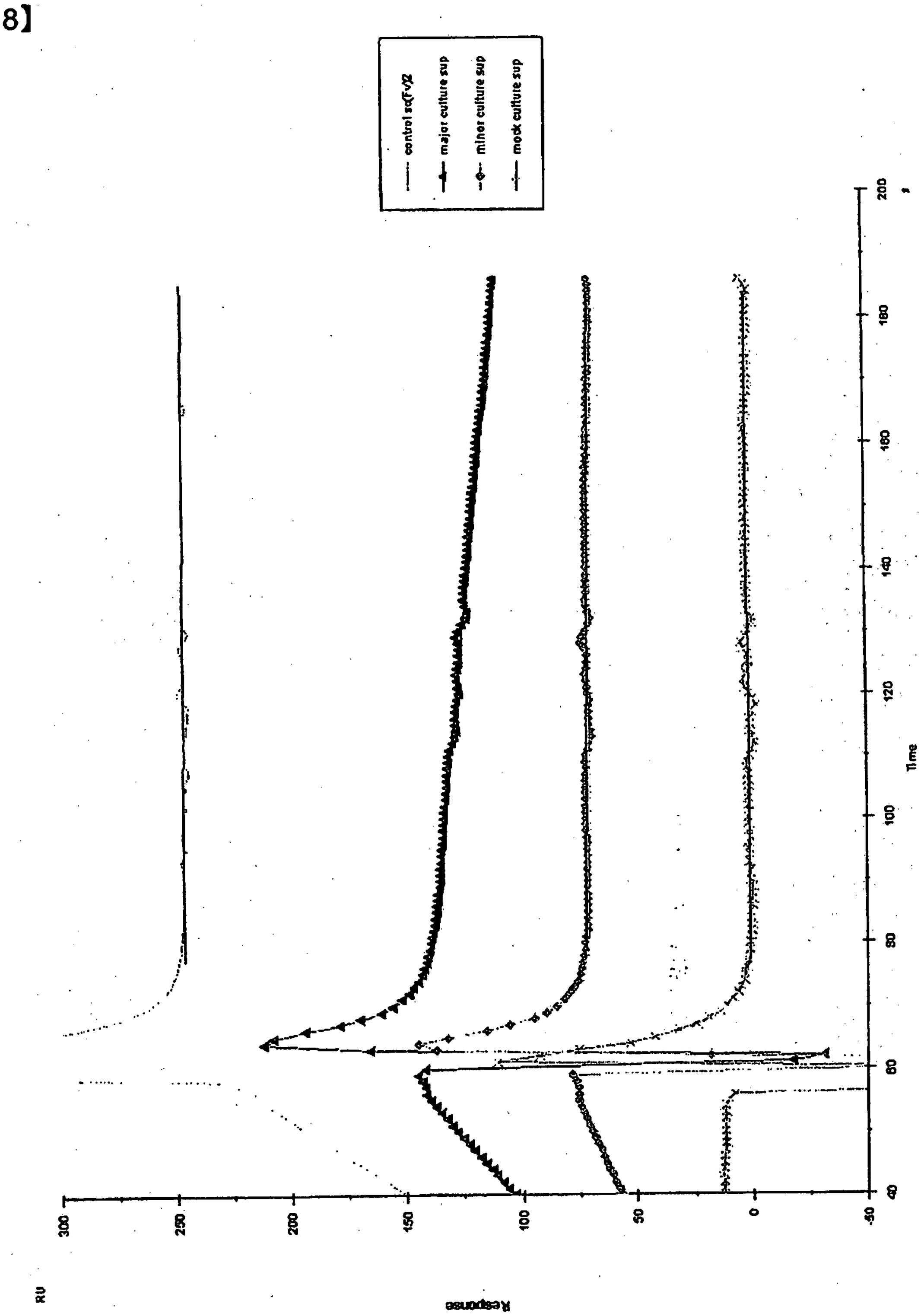
control sc(Fv)2
major culture sup
minor culture sup
mock culture sup
RU
Response
300
250
200
150
100
50
0
-50
Time
40
60
80
100
120
140
160
180
200

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5168062 A
- WO 2001066737 A
- JP 2006224657 A


### Non-patent literature cited in the description


- *J. Mol. Biol.,* 1996, vol. 262, 407-412
- *Cell Technology,* 1986, vol. 5, 212-221
- *J. Biosci. Bioeng.,* 2001, vol. 91 (1), 53-7
- *Blood,* 2005, vol. 105 (2), 562-6
- *J. Immunol. Method,* 1998, vol. 219, 119-29
- **HUSTON et al.** *PNAS USA,* 1988, vol. 85 (16), 5879-5883
- **HEE et al.** *Protein Expression and Purification,* 2002, vol. 25 (2), 270-282
- **TURNER et al.** *Journal of Immunological Methods,* 1997, vol. 205 (1), 43-54
- **HUSTON et al.** *Methods in Enzymology,* 1991, vol. 203, 46-88
- **TAKKINEN et al.** *Protein Engineering,* 1991, vol. 4 (7), 837-841
- **VERMA et al.** *Journal of Immunological Methods,* 1998, vol. 216 (1-2), 165-181
- **ARBABI-GHAHROUDI et al.** *Cancer and metastasis Reviews,* 2005, vol. 24 (4), 501-519
- **TRINH et al.** *Molecular Immunology,* 2004, vol. 40 (10), 717-722
- Molecular Cloning, A Laboratory Manual. Cold Spring Harbor Laboratory Press, 1989, vol. 1, 2, 3
- **KNAPPIK et al.** *Protein Eng.,* 1995, vol. 8 (1), 81-9
- *Nature,* 1989, vol. 341, 544-6
- *Proc. Natl. Acad. Sci. USA.,* 1988, vol. 85 (16), 5879-83
- *Proc. Natl. Acad. Sci. USA.,* 1993, vol. 90, 6444-8
- **HUDSON et al.** *J. Immunol. Methods,* 1999, vol. 231, 177-189
- *J. Immunol. Methods,* 1997, vol. 205 (1), 43-54
- *Prot. Eng.,* 1998, vol. 11 (5), 405-410
- *Mol. Immunol.,* 2004, vol. 40, 717-22
- *Microbiol. Rev.,* 1990, vol. 54 (2), 198-210
- *Protein Science,* 2005, vol. 14, 617-25
- *Biotechniques,* 1992, vol. 12 (6), 864-9
- *Gene,* 1995, vol. 152, 271-5
- *Methods in Enzymol.,* 1983, vol. 100, 468-500
- *Nucleic Acids Res.,* 1984, vol. 12, 9441-56
- *Methods in Enzymol.,* 1987, vol. 154, 350-67
- *Proc. Natl. Acad. Sci. USA.,* 1985, vol. 82, 488-492
- *Methods in Enzymol.,* 1988, vol. 85, 2763-6
- *Biochem. Biophys. Res. Commun.,* 1993, vol. 192 (2), 720-7
- *Nuc. Acids Res.,* 1990, vol. 18 (17), 5322
- *J. Gen. Microbiol.,* 1984, vol. 130 (10), 2527-34
- *Proc. Natl. Acad. Sci.. USA,* 1987, vol. 84 (5), 1177-81
- *DNA,* 1988, vol. 7 (3), 219-25
- *Proc. Natl. Acad. Sci. USA.,* 1996, vol. 93 (3), 1320-4
- *Cell,* 1984, vol. 37, 1053-1062
- *Nature,* 1992, vol. 357, 700-702
- *Nature,* 1979, vol. 277, 108
- *Oncogene,* 1992, vol. 7 (10), 2081-3
- *Nuc. Acids. Res.,* 1990, vol. 18, 5322
- *FASEB J.,* 1992, vol. 6, 2422-7
- *Science,* 1988, vol. 240, 1041-3
- *Proc. Natl. Acad. Sci. USA.,* 1975, vol. 72 (3), 784-8
- *J. Bacteriol.,* 1987, vol. 169, 4379
- **ED HARLOW ; DAVID LANE.** Antibodies: A Laboratory Manual. Cold Spring Harbor Laboratory, 1988
- Remington's Pharmaceutical Science. Mark. Publishing Company
- **K. NISHI ; M. TAKAI ; K. MORIMUNE ; H. OHKAWA.** Molecular and Immunochemical Characteristics of Monoclonal and Recombinant Antibodies Specific to Bisphenol A. *Bioscience, Biotechnology and Biochemistry,* 2003, vol. 67 (6), 1358-1367
- **KIKUCHI et al.** *J. Biosci. Bioeng.,* 2005, vol. 100, 311-317
- *Biochem. Biophys. Res. Com.,* 2004, vol. 315, 912-918